# EUROPEAN PATENT APPLICATION

(11) **EP 4 700 117 A2**
(43) Date of publication of application: **25.02.2026**
(21) Application number: 25214289.8
(22) Date of filing: 17.04.2019
(51) Int. Cl.: C12N 5/0775

(54) **COMPOSITIONS AND METHODS FOR THE TREATMENT OF ISCHEMIA AND CARDIOMYOPATHY**

(30) Priority: 18.04.2018 US 201862659667 P
(62) Divisional of application: 19789191.4
(71) Applicant: Summa Health, Akron, OH 44304 (US); Northeast Ohio Medical University, Rootstown, Ohio 44272 (US)
(72) Inventor: The designation of the inventor has not yet been filed
(74) Representative: Tomkins & Co

(57) **Abstract**

Provided herein are methods to promote tissue healing, to treat tissue injury or to treat a condition related to tissue injury comprising administering to a subject in need thereof an effective amount of one or both of: a CAMKKI polypeptide or an equivalent thereof; or an SDF-1 polypeptide or an equivalent thereof, in the presence of CXCR4 polypeptide expression, wherein the effective amount is an amount that induces exosome production, secretion and/or function in the subject. Recombinant stem cells are also provided that overexpress one or both of a CAMKKI protein or polypeptide and/or an SDF-1 protein or polypeptides. The stem cells and products produced from them are useful to treat ischemic or inflammatory conditions as well as to promote tissue healing.

## Description

### CROSS REFERENCE TO RELATED APPLICATIONS

This application claims priority under 35 U.S.C. § 119(e) to U.S. Provisional Application No. 62/659,667, filed April 18, 2018, the contents of which are hereby incorporated by reference in its entirety.

### BACKGROUND

Ischemia is a condition wherein the blood flow is completely obstructed or considerably reduced in localized parts of the body, resulting in anoxia, reduced supply of substrates and accumulation of metabolites. Prolonged ischemia results in atrophy, denaturation, apoptosis, and necrosis of affected tissues.

Inflammation is a protective response that is intended to eliminate an initial cause of an injury, as well as necrotic cells/tissues resulting from the injury. In some diseases, such as arthritis, however, inflammation occurs in the absence of an injury. Prolonged inflammation can cause tissue destruction, fibrosis, and/or necrosis.

### SUMMARY

Provided herein are methods to promote tissue healing, to treat tissue injury, or to treat a condition related to tissue injury, comprising, or alternatively consisting essentially of, or yet further consisting of administering to a subject in need thereof an effective amount of one or both of:
a. a CAMKK1 polypeptide or an equivalent thereof; or
b. an SDF-1 polypeptide or an equivalent thereof, in the presence of CXCR4 polypeptide expression,
wherein the effective amount is an amount that induces exosome production, secretion and/or function in the subject.

In one aspect, the CAMKK 1 polypeptide can comprise, or alternatively consist essentially of, or yet further consist of SEQ ID NO: 1 or 2, or an equivalent of each thereof. In another aspect, the SDF-1 polypeptide may comprise, or alternatively consist essentially of, or yet further consist of SEQ ID NO: 3 or 4, or an equivalent of each thereof. In a further aspect, the CXRC4 polypeptide can comprise, or alternatively consist essentially of, or yet further consist of SEQ ID NO 5, or an equivalent thereof.

The methods described above may further comprise, or alternatively consist essentially of, or yet further consist of determining the level of circulating exosomes in a sample isolated from the patient prior to administration. In another aspect, the methods may further comprise, or alternatively consist essentially of, or yet further consist of determining the level of circulating exosomes in a sample isolated from the patient after administration. The effective amount can be determined from the level of circulating exosomes in the sample by the treating physician, health professional or treating veterinarian, or based on historical clinical information and data.

Further disclosed herein is a recombinant stem cell that overexpresses one or both of:
a. a CAMKK1 polypeptide or an equivalent thereof; or
b. an SDF-1 polypeptide or an equivalent thereof, in the presence of CXCR4 polypeptide expression.
In one aspect, the overexpression is by expression of a CAMKK1 polypeptide that comprises, or alternatively consists essentially of, or yet further consists of SEQ ID NO: 1 or 2, or by expression of an SDF-1 polypeptide that comprises, or alternatively consists essentially of, or yet further consists of SEQ ID NO: 3 or 4, and the CXCR4 polypeptide comprises, or alternatively consists essentially of, or yet further consists of SEQ ID NO: 5, or an equivalent of each thereof.

This disclosure further provides a recombinant stem cell that overexpresses one or both of a CAMKK1 polypeptide or protein and/or an SDF-1 polypeptide or protein, or an equivalent of each thereof. In one aspect, the overexpression of CAMKK1 and/or SDF-1 is in the presence of Akt expression or optionally overexpression. In another aspect, the overexpression of SDF-1 is the presence of CXCR4 expression, or optionally overexpression. The CXCR4 polypeptide expression can be endogenous or induced by exogenous expression of CXCR4 polynucleotides. The stem cell can be embryonic, an induced pluripotent stem cell (iPSC) or a somatic (adult) and can be of any particular type, including for example, adipose, mesenchymal, or neuronal. In another aspect, the stem cell is a mesenchymal stem cell such as a human mesenchymal stem cell.

In another aspect, the stem cell is of any type or any species, e.g., a mammalian stem cell such as a human stem cell, in one aspect a mesenchymal stem cell or a CD34+ expressing stem cell. Differentiated cells from the recombinant stem cells overexpressing one or both polypeptides and proteins, are further provided herein. The cells are useful in the therapies and methods as disclosed herein.

A non-limiting example of the polynucleotide encoding the CAMKK1 polypeptide or protein comprises, or alternatively consists essentially of, yet further consists of the polypeptide encoding SEQ ID NO.: 1 or 2, or their equivalents. A polynucleotide encoding this sequence also is disclosed at ncbi.nlm.nih.gov/nuccore/NM_032294 (last accessed on April 13, 2018). A non-limiting example of the polynucleotide encoding the SDF-1 polypeptide or protein comprises, or alternatively consists essentially of, yet further consists of the sequence encoding the polypeptide of SEQ ID NO.: 4 or its equivalent, e.g., SEQ ID NO: 3, or its equivalent. These polynucleotides to be inserted in, and when within the stem cells can further comprise, or alternatively consist essentially of, or yet further consist of, a promoter and/or an enhancer sequence operatively linked to the polynucleotide or its equivalent. These polynucleotides can be contained within a vector, e.g., a prokaryotic or a eukaryotic vector, such as a plasmid or a viral vector. Methods to determine if the cell expresses the polypeptide or protein at the appropriate level are known in the art and include for example PCR technology and hybridization techniques (Northern and Southern techniques).

Methods to prepare such cells are known in the art, e.g., by inserting the gene encoding the protein or polypeptide into the stem cell and culturing the cell under suitable conditions. Expression of gene can be determined using methods known in the art, e.g., PCR and/or ELISA technologies.

Also provided herein are isolated cell culture medium produced by the stem cells as described above and the exosomes produced by the stem cells. The exosome compositions can be substantially purified or highly purified from the cells or cell culture media.

The above noted stem cells, cell culture media and exosomes can be combined with carriers to prepare compositions. Non-limiting examples of carriers include a pharmaceutically acceptable carrier or a biocompatible matrix, and can optionally further comprise a preservative, stabilizer and/or a cryoprotectant. In a further aspect, the compositions are lyophilized compositions and comprise the exosomes.

Also provided herein are methods to induce exosome secretion and/or function in a stem cell, the method comprising, or alternatively consisting essentially of, or yet further consisting of, upregulating the expression of CAMKK1 polynucleotide or protein or an equivalent of each thereof in the stem cell. In one aspect, the overexpression of CAMKK1 is in the presence of Akt expression or optionally overexpression. The stem cell can be embryonic, an induced pluripotent stem cell (iPSC) or a somatic (adult) stem cell and can be of any particular type, including for example, adipose, mesenchymal, or neuronal for example. In another aspect, the stem cell is a mesenchymal stem cell. In another aspect, the stem cell is of any type or any species, e.g., a mammalian stem cell such as a human stem cell. Differentiated cells produced from these stem cells are further provided herein.

A non-limiting example of the polynucleotide encoding the CAMKK1 polypeptide or protein comprises, or alternatively consists essentially of, yet further consists of the sequence encoding SEQ ID NOs: 1 and 2, or their equivalent. A polynucleotide encoding sequence also is disclosed at ncbi.nlm.nih.gov/nuccore/NM_032294 (last accessed on April 13, 2018). The polynucleotides in the stem cells can further comprise, or alternatively consist essentially of, or yet further consist of, a promoter and/or an enhancer sequences operatively linked to the polynucleotide or its equivalent. These polynucleotides can be further contained within a vector, e.g., a prokaryotic or a eukaryotic vector, such as a plasmid or a viral vector. Methods to determine if the cell expresses the polypeptide or protein at the appropriate level are known in the art and include PCR.

The methods can further comprise culturing the cell for an effective amount of time for the cells to export exosomes into the culture media and then optionally separating the cells and/or the exosomes from the culture media.

Also provided are the cells, the culture media and the exosomes prepared by the methods. In a further aspect, the method further comprises isolating a population of exosomes from the culture media or the stem cells.

As noted above, stem cells, cell culture media and exosomes can be combined with carriers to prepare compositions. Non-limiting examples of carriers include a pharmaceutically acceptable carrier or a biocompatible matrix, and can optionally further comprising a preservative, stabilizer and/or a cryoprotectant. In a further aspect, the compositions are lyophilized compositions and comprise the exosomes.

Yet further provided is a method to induce exosome secretion and function in a stem cell, comprising, or alternatively consisting essentially of, or yet further consisting of, upregulating the expression of an SDF-1 polypeptide or protein or an equivalent of each thereof in the stem cell, and wherein the overexpression is in the presence of CXCR4 expression. In one aspect, the overexpression of SDF-1 is in the presence of Akt expression or optionally overexpression. Also provided is a method to induce exosome secretion and function in a stem cell, comprising, or alternatively consisting essentially of, or yet further consisting of, upregulating the expression of an SDF-1 polypeptide or protein or an equivalent of each thereof in the stem cell, and wherein the overexpression is in the presence of Akt expression or optionally overexpression. In one aspect, the overexpression of SDF-1 is in the presence of CXCR4 expression or optionally overexpression. The stem cell can be embryonic, an induced pluripotent stem cell (iPSC) or a somatic (adult) stem cell and can be of any particular type, including for example, adipose, mesenchymal, or neuronal for example. In another aspect, the stem cell is a mesenchymal stem cell. In another aspect, the stem cell is of any type or any species, e.g., a mammalian stem cell such as a human stem cell. Differentiated cells produced from the stem cells are further provided herein.

A non-limiting example of the polynucleotide encoding the SDF-1 polypeptide or protein comprises, or alternatively consists essentially of, yet further consists of the sequence of SEQ ID NO: 3, or its equivalent. These polynucleotides in the stem cells can further comprise, or alternatively consist essentially of, or yet further consist of, a promoter and/or an enhancer sequences operatively linked to the polynucleotide or its equivalent. These polynucleotides can be contained within a vector, e.g., a prokaryotic or a eukaryotic vector, such as a plasmid or a viral vector. Methods to determine if the cell expresses the polypeptide or protein at the appropriate level are known in the art and include PCR, Northern hybridization and Southern hybridization.

The methods can further comprise culturing the cell for an effective amount of time for the cells to export exosomes into the culture media and then optionally separating the cells and/or exosomes from the culture media, as well as differentiating the cells into linage specific cell types.

Also provided are the cells, the culture media and the exosomes prepared by the methods. In a further aspect, the method further comprises isolating a population of exosomes from the culture media or the stem cells.

As noted above, stem cells, cell culture media and exosomes can be combined with carriers to prepare compositions. Non-limiting examples of carriers include a pharmaceutically acceptable carrier or a biocompatible matrix, and can optionally further comprising a preservative, a stabilizer and/or a cryoprotectant. In a further aspect, the compositions are lyophilized compositions and comprise the exosomes.

Therapeutic methods also are provided. In one aspect, a method to treat one or more of: ischemia, cardiomyopathy and related disorders, or to promote tissue healing are provided. The methods comprise, or alternatively consist essentially of, or yet further consist of, administering to a subject in need thereof an effective amount one or more of a stem cell as described herein, the isolated culture media as described herein, the exosomes, the populations as described herein, or the compositions as described herein, and wherein the overexpressed polypeptide or protein is the CAMKK1 polypeptide or protein, or an equivalent of thereof. Further provided herein are methods to treat one or more of: an ischemic condition, cardiomyopathy and related disorders or to promote tissue healing, comprising, or alternatively consisting essentially of administering to a subject in need thereof an effective amount of one or more of: the exosome population of this disclosure or the composition described herein. The cells, culture media, or exosomes can be allogeneic or autologous to the subject being treated. In one aspect, the administration is local or systemic. In another aspect, the administration is systemic and is distal to the site of action, e.g., the heart.

### BRIEF DESCRIPTION OF THE DRAWINGS

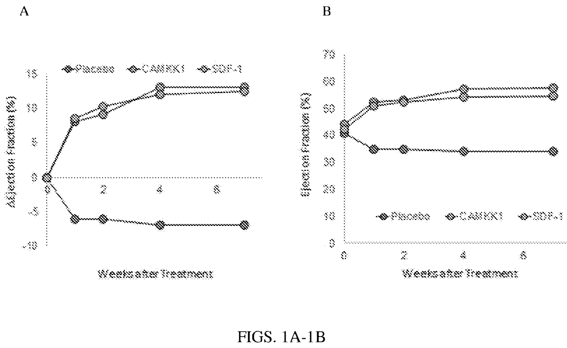

**FIGS. 1A-1B** shows that SDF-1 and CAMKK1 individually had significant and equivalent effects resulting in increased ejection fraction compared to placebo. The combination of SDF-1 and CAMKK1 did not result in any greater increase in cardiac function than either of the molecules alone.

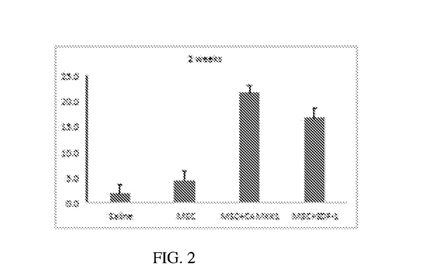

**FIG. 2** shows that a significant effect of SDF-1 and CAMKK1 is the enhancement of exosome release and function into the conditioned media.

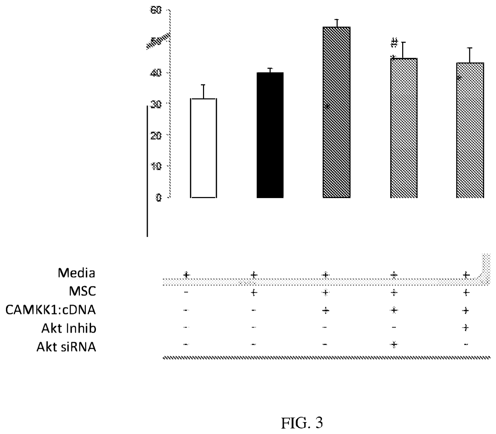

**FIG. 3** shows that the effects of SDF-1 requires an autocrine or paracrine loop to induce Akt phosphorylation, while the CAMKK1 effect is direct.

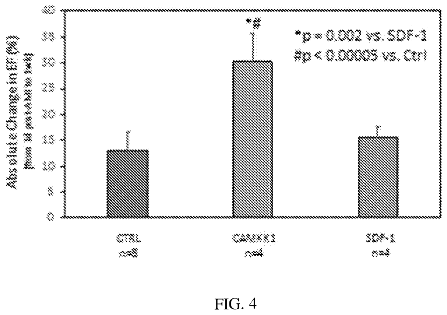

**FIG. 4** shows that the CAMKK1 could be injected into normal tissue remote from the heart and still improve cardiac function. Conversely, since SDF-1 required binding to CXCR4 to induce Akt phosphorylation, that SDF-1 injected into normal tissue remote from the heart would not result in significant improvement of cardiac function.

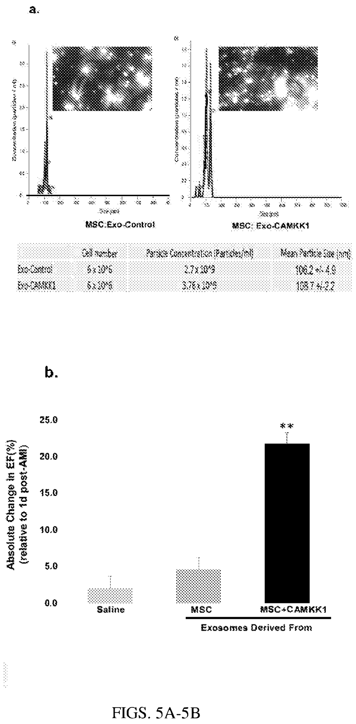

**FIG. 5A-5B** show nanosight quantification of exosome number and size from control MSC and those over-expressing CAMKK1. **FIG. 5A****,** is a representative screen shot of the NTA videos for Exo-control and Exo-CAMKK1. Exosomes-Control showed a considerable number of particles (2.7 × 109 ± 4.9 particles/ml). However, the particle concentration of the Exo-CAMKK1 was higher than control medium (3.76 × 109 ± 2.2 particles/ml, p < 0.05). NTA estimated the size of the Exosomes between 10nm - 1000nm. **FIG. 5B****,** Ejection fraction 2 weeks after left anterior descending ligation. Data are means ± SEM (n = 4-8 per group). *, p < .05 CAMKK1 group versus MSC group; Abbreviations; MSC, mesenchymal stem cells; CAMKK1, Calcium Calmodulin Kinases Kinases 1.

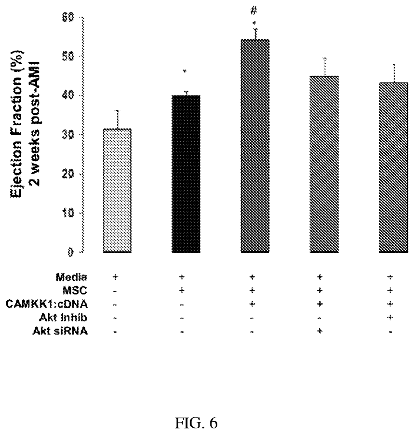

**FIG. 6** shows the MSC effects of CAMKK1 on cardiac function requires the phosphorylation of akt. The ejection fraction as a measure of cardiac function, was measured 2 weeks after left anterior descending ligation in hearts injected the conditioned media of 2 million MSC were collected and injected into the heart of animals immediately after inducing an anterior wall myocardial infarction. In two treatments MSC were treated with one of two Akt inhibitors and the presence of either Akt inhibitor decreased the benefits of the CAMKK1 over-expressing cells to that of control MSC. Data are means ± SEM (n = 4-8 per group) *, p < .05 cDNA-CAMKK1 versus MSC control group. Abbreviations: AKT, protein kinase B, MSC, mesenchymal stem cells.

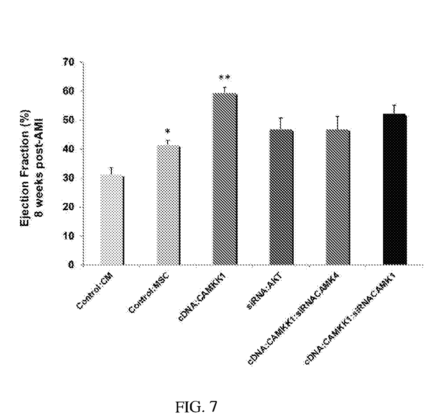

**FIG. 7** shows the ejection fraction as a measure of cardiac function, was measured 2 weeks after left anterior descending ligation in hearts injected Exo-MSC control, Exo-MSC over-expressing CAMKK1, Exo overexpressing, siRNA AKT. It is shown that CAMKK1 is a kinase and directly phosphorylates akt resulting in the release of exosomes from the CAMKK1 over-expressing cells, that the CAMKK1 could be injected into normal tissue remote from the heart and still improve cardiac function.

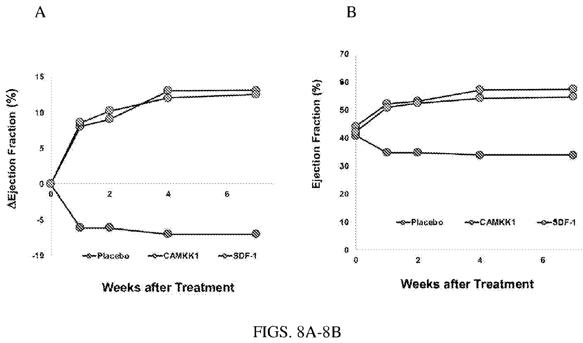

**FIGS. 8A-8B** shows that CAMKK1 had an effect in chronic heart failure, and how this compared to SDF-1. CAMKK1 injections at the time of AMI, resulted in improved ejection fraction measurements after 1 and 6 weeks. It was assumed that SDF-1 worked through its chemokine effect and resulted in improvement in cardiac function through the homing of tissue specific and bone marrow derived stem cells. CAMKK1 does not induce stem cell homing. That CAMKK1 and SDF-1 had equivalent effects on cardiac function, and the combination of the two did not further enhance their effect, that led to a series of studies trying to define a common pathway through which SDF-1 and CAMKK1 might work. CAMMKK1 levels 57.4%, 53.2%, 44.2% and 40.1% at placebo, and SDF-1 levels 54.7%, 52.5%,50.4% and 40.2% at placebo 1 day and 6 days post-AMI). Data are presented as mean ± SEM n=5-8 animals per group (**, p < .05 compared with saline control).

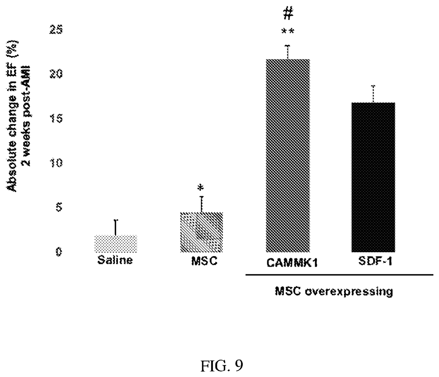

**FIG. 9** shows overexpression of CAMKK1 and SDF-1 enriches the amount of exosomes in MSCs. Cardiac function (ejection fraction) 2 weeks after AMI, CAMKK1 and SDF-1 produced a significant improvement in EF after 2 weeks post- AMI. The significant effect of SDF-1 and CAMKK1 is the enhancement of exosome release and function into the conditioned media. The cardiac function was significantly improved by CAMKK1 compared with Saline control and SDF-1 injections. CAMKK1 led to a significant improvement in EF (21.8% ± 2.5, p < .01) and SDF-1 (16.8% ± 2.7, p > .05) compared with Saline control group (1.9% ± 0.2) after 2 weeks post-AMI. Data are mean ± SEM (n = 7-8 per each group). Cardiac function improvement induced by CAMKK1 overexpression in MSC correlates with increased vascular density in cardiac border zone after 2 weeks post-AMI.

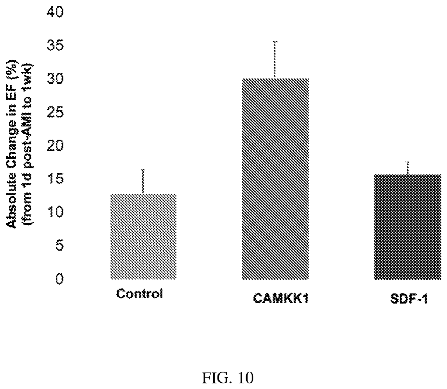

**FIG. 10** shows heart function and echocardiography results 14 days post AMI. The animals underwent ligation of LAD and immediately after the chest was closed, the animals receiving CAMKK1 plasmid or SDF-1 plasmid or saline, was injected into the thigh of the animal. One week later, the cardiac function of the animals injected with CAMKK1 was significantly increased compared to saline, but the heart function in SDF-1 treated animals was not. CAMKK1 (30.3% ± 1.5, p < .01) and SDF-1 (15.7% ± 3.7, p > .05) compared with Saline control group (12.9% = 1.8) after 1 week later post-AMI. Data are mean ± SEM (n = 7-8 per each group).

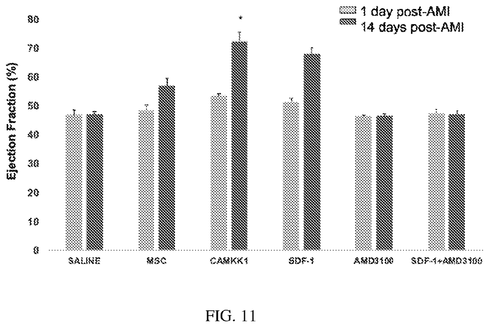

**FIG. 11** shows the effects of CAMKK1 and SDF-1 overexpression on the function of MSC-exosomes and cardiac function (ejection fraction). Exosomes were purified from the conditioned media (CM) of 2 million control MSC, and MSC over-expressing either SDF-1(with/without AMD3100) or CAMKK1. The isolated exosomes were then injected into the heart of a rat immediately after AMI. Compared with the Saline control group 2 weeks after AMI, significant cardiac function improvement was found in CAMKK1 (21.8%) and SDF-1 groups (16.8 %). The benefits were lost in the SDF-1+AMD3100 group. Data are mean ± SEM (n = 7-8 per each group).

### DETAILED DESCRIPTION

### Definitions

The following abbreviations are used throughout the disclosure: CAMKK1 (calcium/calmodulin-dependent protein kinase kinase 1); MSC (mesenchymal stem cells); and stromal cell-derived factor 1 alpha (SDF-1, (also known as CXCL12, Pre-B cell growth-stimulating factor, PBSF, hIRH, chemokine (C-X-C motif) ligand 12, SDF1, SDF1A, TPAR1, SCYB12, SDF-1a, TLSF-a)), C-X-C chemokine receptor type 4 (CXCR-4) is also known as fusin or CD184 (CXCR4).

Unless defined otherwise, all technical and scientific terms used herein have the same meanings as commonly understood by one of ordinary skill in the art to which this disclosure belongs. Although any methods and materials similar or equivalent to those described herein can be used in the practice or testing of the present disclosure, the preferred methods, devices, and materials are now described. All technical and patent publications cited herein are incorporated herein by reference in their entirety. Nothing herein is to be construed as an admission that the disclosure is not entitled to antedate such disclosure by virtue of prior disclosure.

The practice of the present disclosure will employ, unless otherwise indicated, conventional techniques of tissue culture, immunology, molecular biology, microbiology, cell biology and recombinant DNA, which are within the skill of the art. See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR 1: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Technique, 5th edition; Gait ed. (1984) Oligonucleotide Synthesis; U.S. Patent No. 4,683,195; Hames and Higgins eds.

(1984) Nucleic Acid Hybridization; Anderson (1999) Nucleic Acid Hybridization; Hames and Higgins eds. (1984) Transcription and Translation; Immobilized Cells and Enzymes (IRL Press (1986)); Perbal (1984) A Practical Guide to Molecular Cloning; Miller and Calos eds. (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); Herzenberg et al. eds (1996) Weir's Handbook of Experimental Immunology; Manipulating the Mouse Embryo: A Laboratory Manual, 3rd edition (Cold Spring Harbor Laboratory Press (2002)); Sohail (ed.) (2004) Gene Silencing by RNA Interference: Technology and Application (CRC Press).

All numerical designations, e.g., pH, temperature, time, concentration, and molecular weight, including ranges, are approximations which are varied ( + ) or ( - ) by increments of 1.0 or 0.1, as appropriate. It is to be understood, although not always explicitly stated that all numerical designations are preceded by the term "about". It also is to be understood, although not always explicitly stated, that the reagents described herein are merely exemplary and that equivalents of such are known in the art.

As used in the specification and claims, the singular form "a," "an" and "the" include plural references unless the context clearly dictates otherwise.

As used herein, the term "comprising" is intended to mean that the compositions and methods include the recited elements, but do not exclude others. "Consisting essentially of" when used to define compositions and methods, shall mean excluding other elements of any essential significance to the combination when used for the intended purpose. Thus, a composition consisting essentially of the elements as defined herein would not exclude trace contaminants or inert carriers. "Consisting of" shall mean excluding more than trace elements of other ingredients and substantial method steps. Embodiments defined by each of these transition terms are within the scope of this disclosure.

The terms "polynucleotide", "nucleic acid" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this disclosure that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present disclosure.

A polynucleotide or polynucleotide region (or a polypeptide or polypeptide region) has a certain percentage (for example, 70%, 75%, 80%, 85%, 90%, 95%, 98% or 99%) of "sequence identity" to another sequence means that, when aligned, that percentage of bases (or amino acids) are the same in comparing the two sequences. This alignment and the percent homology or sequence identity can be determined using software programs known in the art, for example those described in Ausubel et al. eds. (2007) Current Protocols in Molecular Biology. Preferably, default parameters are used for alignment. One alignment program is BLAST, using default parameters. In particular, programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: http://www.ncbi.nlm.nih.gov/cgi-bin/BLAST.

An equivalent or biological equivalent nucleic acid, polynucleotide or oligonucleotide or peptide is one having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to the reference nucleic acid, polynucleotide, oligonucleotide or peptide.

The term "amplification of polynucleotides" includes methods such as PCR, ligation amplification (or ligase chain reaction, LCR) and amplification methods. These methods are known and widely practiced in the art. See, e.g., U.S. Pat. Nos. 4,683,195 and 4,683,202 and Innis et al., 1990 (for PCR); and Wu et al. (1989) Genomics 4:560-569 (for LCR). In general, the PCR procedure describes a method of gene amplification which is comprised of (i) sequence-specific hybridization of primers to specific genes within a DNA sample (or library), (ii) subsequent amplification involving multiple rounds of annealing, elongation, and denaturation using a DNA polymerase, and (iii) screening the PCR products for a band of the correct size. The primers used are oligonucleotides of sufficient length and appropriate sequence to provide initiation of polymerization, i.e. each primer is specifically designed to be complementary to each strand of the genomic locus to be amplified.

Reagents and hardware for conducting PCR are commercially available. Primers useful to amplify sequences from a particular gene region are preferably complementary to, and hybridize specifically to sequences in the target region or its flanking regions. Nucleic acid sequences generated by amplification may be sequenced directly. Alternatively, the amplified sequence(s) may be cloned prior to sequence analysis. A method for the direct cloning and sequence analysis of enzymatically amplified genomic segments is known in the art.

A "gene" refers to a polynucleotide containing at least one open reading frame (ORF) that is capable of encoding a particular polypeptide or protein after being transcribed and translated.

The term "express" refers to the production of a gene product.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in a eukaryotic cell.

A "gene product" or alternatively a "gene expression product" refers to the amino acid (e.g., peptide or polypeptide) generated when a gene is transcribed and translated.

"Under transcriptional control" is a term well understood in the art and indicates that transcription of a polynucleotide sequence, usually a DNA sequence, depends on its being operatively linked to an element which contributes to the initiation of, or promotes, transcription. "Operatively linked" intends the polynucleotides are arranged in a manner that allows them to function in a cell. In one aspect, this disclosure provides promoters operatively linked to the downstream sequences, e.g., CAMKK1, SDF-1etc.

The term "encode" as it is applied to polynucleotides refers to a polynucleotide which is said to "encode" a polypeptide if, in its native state or when manipulated by methods well known to those skilled in the art, it can be transcribed and/or translated to produce the mRNA for the polypeptide and/or a fragment thereof. The antisense strand is the complement of such a nucleic acid, and the encoding sequence can be deduced therefrom.

A "probe" when used in the context of polynucleotide manipulation refers to an oligonucleotide that is provided as a reagent to detect a target potentially present in a sample of interest by hybridizing with the target. Usually, a probe will comprise a detectable label or a means by which a label can be attached, either before or subsequent to the hybridization reaction. Alternatively, a "probe" can be a biological compound such as a polypeptide, antibody, or fragments thereof that is capable of binding to the target potentially present in a sample of interest.

"Detectable labels" or "markers" include, but are not limited to radioisotopes, fluorochromes, chemiluminescent compounds, dyes, and proteins, including enzymes. Detectable labels can also be attached to a polynucleotide, polypeptide, antibody or composition described herein.

A "primer" is a short polynucleotide, generally with a free 3' -OH group that binds to a target or "template" potentially present in a sample of interest by hybridizing with the target, and thereafter promoting polymerization of a polynucleotide complementary to the target. A "polymerase chain reaction" ("PCR") is a reaction in which replicate copies are made of a target polynucleotide using a "pair of primers" or a "set of primers" consisting of an "upstream" and a "downstream" primer, and a catalyst of polymerization, such as a DNA polymerase, and typically a thermally-stable polymerase enzyme. Methods for PCR are well known in the art, and taught, for example in MacPherson et al. (1991) PCR 1: A Practical Approach (IRL Press at Oxford University Press). All processes of producing replicate copies of a polynucleotide, such as PCR or gene cloning, are collectively referred to herein as "replication." A primer can also be used as a probe in hybridization reactions, such as Southern or Northern blot analyses. Sambrook and Russell (2001), infra.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Hybridization reactions can be performed under conditions of different "stringency". In general, a low stringency hybridization reaction is carried out at about 40 °C in 10 x SSC or a solution of equivalent ionic strength/temperature. A moderate stringency hybridization is typically performed at about 50 °C in 6 x SSC, and a high stringency hybridization reaction is generally performed at about 60 °C in 1 x SSC. Additional examples of stringent hybridization conditions include: low stringency of incubation temperatures of about 25°C to about 37°C; hybridization buffer concentrations of about 6x SSC to about 10x SSC; formamide concentrations of about 0% to about 25%; and wash solutions from about 4x SSC to about 8x SSC. Examples of moderate hybridization conditions include: incubation temperatures of about 40°C to about 50°C; buffer concentrations of about 9x SSC to about 2x SSC; formamide concentrations of about 30% to about 50%; and wash solutions of about 5x SSC to about 2x SSC. Examples of high stringency conditions include: incubation temperatures of about 55°C to about 68°C; buffer concentrations of about 1x SSC to about 0.1x SSC; formamide concentrations of about 55% to about 75%; and wash solutions of about 1x SSC, 0.1x SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed. Hybridization reactions can also be performed under "physiological conditions" which is well known to one of skill in the art. A non-limiting example of a physiological condition is the temperature, ionic strength, pH and concentration of Mg²⁺ normally found in a cell.

When hybridization occurs in an antiparallel configuration between two single-stranded polynucleotides, the reaction is called "annealing" and those polynucleotides are described as "complementary". A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to form hydrogen bonding with each other, according to generally accepted base-pairing rules.

The term "propagate" or "expand" means to grow a cell or population of cells. The term "growing" also refers to the proliferation of cells in the presence of supporting media, nutrients, growth factors, support cells, or any chemical or biological compound necessary for obtaining the desired number of cells or cell type.

The term "culturing" refers to the in vitro propagation of cells or organisms on or in media of various kinds. It is understood that the descendants of a cell grown in culture may not be completely identical (i.e., morphologically, genetically, or phenotypically) to the parent cell.

As used herein, the term "vector" refers to a non-chromosomal nucleic acid comprising an intact replicon such that the vector may be replicated when placed within a cell, for example by a process of transformation. Vectors may be viral or non-viral. Viral vectors include retroviruses, adenoviruses, herpesvirus, bacculoviruses, modified bacculoviruses, papovirus, or otherwise modified naturally occurring viruses. Exemplary non-viral vectors for delivering nucleic acid include naked DNA; DNA complexed with cationic lipids, alone or in combination with cationic polymers; anionic and cationic liposomes; DNA-protein complexes and particles comprising DNA condensed with cationic polymers such as heterogeneous polylysine, defined-length oligopeptides, and polyethylene imine, in some cases contained in liposomes; and the use of ternary complexes comprising a virus and polylysine-DNA. Non-limiting examples of plasmid vectors include pHIV, pLVX, pLKO.1, pLemiR, pLV, pUC, pGEM, pSP73, pSP72, pSP64, pBR322 and pCCL plasmids.

A "viral vector" is defined as a recombinantly produced virus or viral particle that comprises a polynucleotide to be delivered into a host cell, either *in vivo, ex vivo* or *in vitro.* Examples of viral vectors include retroviral vectors, lentiviral vectors, adenovirus vectors, adeno-associated virus vectors, alphavirus vectors and the like. Alphavirus vectors, such as Semliki Forest virus-based vectors and Sindbis virus-based vectors, have also been developed for use in gene therapy and immunotherapy. See, Schlesinger and Dubensky (1999) Curr. Opin. Biotechnol. 5:434-439 and Ying, et al. (1999) Nat. Med. 5(7):823-827.

In aspects where gene transfer is mediated by a lentiviral vector, a vector construct refers to the polynucleotide comprising the lentiviral genome or part thereof, and a therapeutic gene. As used herein, "lentiviral mediated gene transfer" or "lentiviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. Retroviruses carry their genetic information in the form of RNA; however, once the virus infects a cell, the RNA is reverse-transcribed into the DNA form which integrates into the genomic DNA of the infected cell. The integrated DNA form is called a provirus. As used herein, lentiviral vector refers to a viral particle capable of introducing exogenous nucleic acid into a cell through a viral or viral-like entry mechanism. A "lentiviral vector" is a type of retroviral vector well-known in the art that has certain advantages in transducing nondividing cells as compared to other retroviral vectors. See, Trono D. (2002) Lentiviral vectors, New York: Spring-Verlag Berlin Heidelberg.

Lentiviral vectors of this disclosure are based on or derived from oncoretroviruses (the sub-group of retroviruses containing MLV), and lentiviruses (the sub-group of retroviruses containing HIV). Examples include ASLV, SNV and RSV all of which have been split into packaging and vector components for lentiviral vector particle production systems. The lentiviral vector particle according to the disclosure may be based on a genetically or otherwise (e.g. by specific choice of packaging cell system) altered version of a particular retrovirus.

A vector particle that is "based on" a particular retrovirus means that the vector is derived from that particular retrovirus. The genome of the vector particle comprises components from that retrovirus as a backbone. The vector particle contains essential vector components compatible with the RNA genome, including reverse transcription and integration systems. Usually these will include gag and pol proteins derived from the particular retrovirus. Thus, the majority of the structural components of the vector particle will normally be derived from that retrovirus, although they may have been altered genetically or otherwise so as to provide desired useful properties. However, certain structural components and in particular the env proteins, may originate from a different virus. The vector host range and cell types infected or transduced can be altered by using different env genes in the vector particle production system to give the vector particle a different specificity.

The term "promoter" refers to a region of DNA that initiates transcription of a particular gene. The promoter includes the core promoter, which is the minimal portion of the promoter required to properly initiate transcription and can also include regulatory elements such as transcription factor binding sites. The regulatory elements may promote transcription or inhibit transcription. Regulatory elements in the promoter can be binding sites for transcriptional activators or transcriptional repressors. A promoter can be constitutive or inducible. A constitutive promoter refers to one that is always active and/or constantly directs transcription of a gene above a basal level of transcription. Non-limiting examples of such include the phosphoglycerate kinase 1 (PGK) promoter; SSFV, CMV, MNDU3, SV40, Efla, UBC and CAGG. An inducible promoter is one which is capable of being induced by a molecule or a factor added to the cell or expressed in the cell. An inducible promoter may still produce a basal level of transcription in the absence of induction, but induction typically leads to significantly more production of the protein. Promoters can also be tissue specific. A tissue specific promoter allows for the production of a protein in a certain population of cells that have the appropriate transcriptional factors to activate the promoter.

An enhancer is a regulatory element that increases the expression of a target sequence. A "promoter/enhancer" is a polynucleotide that contains sequences capable of providing both promoter and enhancer functions. For example, the long terminal repeats of retroviruses contain both promoter and enhancer functions. The enhancer/promoter may be "endogenous" or "exogenous" or "heterologous." An "endogenous" enhancer/promoter is one which is naturally linked with a given gene in the genome. An "exogenous" or "heterologous" enhancer/promoter is one which is placed in juxtaposition to a gene by means of genetic manipulation (i.e., molecular biological techniques) such that transcription of that gene is directed by the linked enhancer/promoter.

The term "isolated" as used herein refers to molecules or biological or cellular materials being substantially free from other materials, e.g., greater than 70%, or 80%, or 85%, or 90%, or 95%, or 98%. In one aspect, the term "isolated" refers to nucleic acid, such as DNA or RNA, or protein or polypeptide, or cell or cellular organelle, or tissue or organ, separated from other DNAs or RNAs, or proteins or polypeptides, or cells or cellular organelles, or tissues or organs, respectively, that are present in the natural source and which allow the manipulation of the material to achieve results not achievable where present in its native or natural state, e.g., recombinant replication or manipulation by mutation. The term "isolated" also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides, e.g., with a purity greater than 70%, or 80%, or 85%, or 90%, or 95%, or 98%. The term "isolated" is also used herein to refer to cells or tissues that are isolated from other cells or tissues and can encompass cultured and/or engineered cells or tissues.

As used herein, "stem cell" defines a cell with the ability to divide for indefinite periods in culture and give rise to specialized cells. At this time and for convenience, stem cells are categorized as somatic (adult), embryonic or induced pluripotent stem cells. A somatic stem cell is an undifferentiated cell found in a differentiated tissue that can renew itself (clonal) and (with certain limitations) differentiate to yield all the specialized cell types of the tissue from which it originated. An embryonic stem cell is a primitive (undifferentiated) cell from the embryo that has the potential to become a wide variety of specialized cell types. Non-limiting examples of embryonic stem cells are the HES2 (also known as ES02) cell line available from ESI, Singapore and the H1 or H9 (also known as WA01) cell line available from WiCell, Madison, WI. Pluripotent embryonic stem cells can be distinguished from other types of cells by the use of markers including, but not limited to, Oct-4, alkaline phosphatase, CD30, TDGF-1, GCTM-2, Genesis, Germ cell nuclear factor, SSEA1, SSEA3, and SSEA4. An induced pluripotent stem cell (iPSC) is an artificially derived stem cell from a non-pluripotent cell, typically an adult somatic cell, produced by inducing expression of one or more stem cell specific genes.

A "parthenogenetic stem cell" refers to a stem cell arising from parthenogenetic activation of an egg. Methods of creating a parthenogenetic stem cell are known in the art. See, for example, Cibelli et al. (2002) Science 295(5556):819 and Vrana et al. (2003) Proc. Natl. Acad. Sci. U S A 100(Suppl. 1)11911-6 (2003).

"Embryoid bodies or EBs" are three-dimensional (3-D) aggregates of embryonic stem cells formed during culture that facilitate subsequent differentiation. When grown in suspension culture, EBs cells form small aggregates of cells surrounded by an outer layer of visceral endoderm. Upon growth and differentiation, EBs develop into cystic embryoid bodies with fluid-filled cavities and an inner layer of ectoderm-like cells.

The term "propagate" means to grow or alter the phenotype of a cell or population of cells. The term "growing" refers to the proliferation of cells in the presence of supporting media, nutrients, growth factors, support cells, or any chemical or biological compound necessary for obtaining the desired number of cells or cell type. In one embodiment, the growing of cells results in the regeneration of tissue. In yet another embodiment, the tissue is comprised of neuronal progenitor cells or neuronal cells.

The term "culturing" refers to the in vitro propagation of cells or organisms on or in media of various kinds. It is understood that the descendants of a cell grown in culture may not be completely identical (i.e., morphologically, genetically, or phenotypically) to the parent cell. By "expanded" is meant any proliferation or division of cells. A "cultured" cell is a cell that has been separated from its native environment and propagated under specific, pre-defined conditions.

"Differentiation" describes the process whereby an unspecialized cell acquires the features of a specialized cell such as a heart, liver, or muscle cell. "Directed differentiation" refers to the manipulation of stem cell culture conditions to induce differentiation into a particular cell type. "Dedifferentiated" defines a cell that reverts to a less committed position within the lineage of a cell. As used herein, the term "differentiates or differentiated" defines a cell that takes on a more committed ("differentiated") position within the lineage of a cell. As used herein, "a cell that differentiates into a mesodermal (or ectodermal or endodermal) lineage" defines a cell that becomes committed to a specific mesodermal, ectodermal or endodermal lineage, respectively. Examples of cells that differentiate into a mesodermal lineage or give rise to specific mesodermal cells include, but are not limited to, cells that are adipogenic, leiomyogenic, chondrogenic, cardiogenic, dermatogenic, hematopoetic, hemangiogenic, myogenic, nephrogenic, urogenitogenic, osteogenic, pericardiogenic, or stromal.

Examples of cells that differentiate into ectodermal lineage include, but are not limited to epidermal cells, neurogenic cells, and neurogliagenic cells.

A "marrow stromal cell" also referred to as "mesenchymal stem cells," or MSC, is a multipotent stem cell that can differentiate into a variety of cell types. Cell types that MSCs have been shown to differentiate into in vitro or in vivo include osteoblasts, chondrocytes, myocytes, and adipocytes. Mesenchyme is embryonic connective tissue that is derived from the mesoderm and that differentiates into hematopoietic and connective tissue, whereas MSCs do not differentiate into hematopoietic cells. Stromal cells are connective tissue cells that form the supportive structure in which the functional cells of the tissue reside. Methods to isolate such cells, propagate and differentiate such cells are known in the technical and patent literature, e.g., U.S. Patent Application Publication Nos. 2007/0224171, 2007/0054399 and 2009/0010895, which are incorporated by reference in their entirety.

A CD34+ cell intends a cell that expresses the cell surface CD34 marker. CD34 derives its name from the cluster of differentiation protocol that identifies cell surface antigens. CD34 was first described on hematopoietic stem cells and functions as a cell-cell adhesion factor. It also mediates the attachment of hematopoietic stem cells to bone marrow extracellular matrix or directly to stromal cells. Clinically, it is associated with the selection and enrichment of hematopoietic stem cells for bone marrow transplants. It is also found on cell types other than hematopoietic cells. Sidney LE, et al. (2014). "Concise review: evidence for CD34 as a common marker for diverse progenitors". Stem Cells. 32 (6): 1380-1389.

As used herein, the term "differentiates or differentiated" defines a cell that takes on a more committed ("differentiated") position within the lineage of a cell. "Dedifferentiated" defines a cell that reverts to a less committed position within the lineage of a cell.

CAMKK1 (calcium/calmodulin-dependent protein kinase kinase 1; human CAMKK1 known by UniProtKB number Q8N5S9) belongs to a calcium-triggered signaling cascade and is involved in a number of cellular processes. CAMKK 1 is a transferase that belongs to Ser/Thr protein kinase family and CamKK subfamily, and is expressed in heart, pancreas, amygdale, hypothalamus, prostate and lung. CAMKK 1 activates CamK1 and CamK4 by phosphorylation of their amino acids Thr(177) and Thr(196), respectively. CAMKK1 activity is itself subjected to regulation by Ca2+/calmodulin; the activity of CAMKK 1 is decreased upon phosphorylation by PKA (cAMP-Dependent Protein Kinase) and increased by incubation with PKA in the presence of Ca(2+)/calmodulin but decreased in its absence. This phosphorylation and inhibition of CAMKK 1 by PKA is involved in modulating the balance between cAMP- and Ca2+-dependent signal transduction pathways. The amino acid sequence of CAMKK 1 is set forth in SEQ ID NO: 1. Truncated and mutated versions, that are examples of equivalents of the CAMKK1 protein are described herein.

SDF-1 (stromal cell-derived factor-1) is small cytokine belonging to the chemokine family that is officially designated Chemokine (C-X-C motif) ligand 12 (CXCL12). It is produced in two forms, SDF-1α/CXCL12a and SDF-1β/CXCL12b, by alternate splicing of the same gene. Chemokines are characterized by the presence of four conserved cysteines, which form two disulfide bonds. The CXCL12 proteins belong to the group of CXC chemokines, whose initial pair of cysteines are separated by one intervening amino acid. CXCL12 is strongly chemotactic for lymphocytes and has been implicated as an important cell coordinator during development. The receptor for this chemokine is CXCR4, which was previously called fusin. This CXCL12-CXCR4 interaction used to be considered exclusive (unlike for other chemokines and their receptors), but recently it was suggested that CXCL12 is also bound by CXCR7 receptor. The gene for CXCL12 is located on human chromosome 10. In human and mouse both CXCL12 and CXCR4 show high identity of sequence: 99% and 90%, respectively. A representative sequence of the human protein is mnakvvvvlv lvltalclsd gkpvslsyrc pcrffeshva ranvkhlkil ntpncalqiv arlknnnrqv cidpklkwiq eylekalnkr fkm (SEQ ID NO.: 4) (reproduced from NCBI Ref. Sequence NP_000600.1, see ncbi.nlm.nih.gov/protein/NP_000600, last accessed on April 12, 2018). A representative encoding polynucleotide is provided in SEQ ID NO: 3, reproduced from NM_199168 (see ncbi.nlm.nih.gov/nuccore/NM_199168, last accessed on April 12, 2018).

CXCR-4 is an alpha-chemokine receptor specific for stromal-derived-factor-1 (SDF-1). CXCR4 is one of several chemokine receptors that HIV can use to infect CD4+T-cells. CXCR4's ligand SDF-1 is known to be important in hematopoietic stem cell homing to the bone marrow and in hematopoietic stem cell quiescence.

A representative CXCR-4 protein sequence (SEQ ID NO: 5) (reproduced from uniprot.org/uniprot/P61073 (last accessed on April 12, 2018)) is:

MEGISIYTSD NYTEEMGSGD YDSMKEPCFR EENANFNKIF LPTIYSIIFL
TGIVGNGLVI LVMGYQKKLR SMTDKYRLHL SVADLLFVIT LPFWAVDAVA
NWYFGNFLCK AVHVIYTVNL YSSVLILAFI SLDRYLAIVH ATNSQRPRKL
LAEKVVYVGV WIPALLLTIP DFIFANVSEA DDRYICDRFY PNDLWVVVFQ
FQHIMVGLIL PGIVILSCYC IIISKLSHSK GHQKRKALKT TVILILAFFA

CWLPYYIGIS IDSFILLEII KQGCEFENTV HKWISITEAL AFFHCCLNPI
LYAFLGAKFK TSAQHALTSV SRGSSLKILS KGKRGGHSSV STESESSSFH

A representative human polynucleotide sequence (SEQ ID NO: 6) (reproduced from ncbi.nlm.nih.gov/nuccore/NG_011587.1?from=5001&to=8807&report=genbank, last accessed on April 12, 2018) is:

1 aacttcagtt tgttggctgc ggcagcaggt agcaaagtga cgccgagggc ctgagtgctc

61 cagtagccac cgcatctgga gaaccagcgg ttaccatgga ggggatcagt gtaagtccag

121 tttcaacctg ctttgtcata aatgtacaaa cgtttgaact tagagcgcag cccctctccg

181 agcgggcaga agcggccagg acattggagg tacccgtact ccaaaaaagg gtcaccgaaa

241 ggagttttct tgaccatgcc tatatagtgc gggtgggtgg ggggggagca ggattggaat

301 ctttttctct gtgagtcgag gagaaacgac tggaaagagc gttccagtgg ctgcatgtgt

361 ctcccccttg agtcccgccg cgcgcggcgg cttgcacgct gtttgcaaac gtaagaacat

421 tctgtgcaca agtgcagaga aggcgtgcgc gctgcctcgg gactcagacc accggtctct

481 tccttgggga agcggggatg tcttggagcg agttacattg tctgaattta gaggcggagg

541 gcggcgtgcc tgggctgagt tcccaggagg agattgcgcc cgctttaact tcggggttaa

601 gcgcctggtg actgttcttg acactgggtg cgtgtttgtt aaactctgtg cggccgacgg

661 agctgtgcca gtctcccagc acagtaggca gagggcggga gaggcgggtg gacccaccgc

721 gccgatcctc tgaggggatc gagtggtggc agcagctagg agttgatccg cccgcgcgct

781 ttgggtttga gggggaaaac cttcccgccg tccgaagcgc gcctcttccc cacggccgcg

841 agtgggtcct gcagttcgag agtttggggt cgtgcagagg tcagcggagt ggtttgacct

901 cccctttgac accgcgcagc tgccagccct gagatttgcg ctccggggat aggagcgggt

961 acggggtgag gggcgggggc ggttaagacc gcacctgggc tgccaggtcg ccgccgcgaa

1021 gactggcagg tgcaagtggg gaaaccgttt ggctctctcc gagtccagtt gtgatgttta

1081 accgtcggtg gtttccagaa accttttgaa accctcttgc tagggagttt ttggtttcct

1141 gcagcggcgc gcaattcaaa gacgctcgcg gcggagccgc ccagtcgctc cccagcaccc

1201 tgtgggacag agcctggcgt gtcgcccagc ggagcccctg cagcgctgct tgcgggcggt

1261 tggcgtgggt gtagtgggca gccgcggcgg cccggggctg gacgacccgg ccccccgcgt

1321 gcccaccgcc tggaggcttc cagctgccca cctccggccg ggttaactgg atcagtggcg

1381 gggtaatggg aagccacccg ggagagtgag gaaatgaaac ttggggcgag gaccacgggt

1441 gcagaccccg ttaccttctc cacccaggaa aatgccccgc tccctaacgt cccaaacgcg

1501 ccaagtgata aacacgagga tggcaagaga cccacacacc ggaggagcgc ccgcttgggg

1561 gaggaggtgc cgtttgttca ttttctgaca ctcccgccca atatacccca agcaccgaag

1621 ggccttcgtt ttaagaccgc attctcttta cccactacaa gttgcttgaa gcccagaatg

1681 gtttgtattt aggcaggcgt gggaaaatta agtttttgcg ctttaggaga atgagtcttt

1741 gcaacgcccc cgccctcccc ccgtgatcct cccttctccc ctcttccctc cctgggcgaa

1801 aaacttctta caaaaagtta atcactgccc ctcctagcag cacccacccc accccccacg

1861 ccgcctggga gtggcctctt tgtgtgtatt ttttttttcc tcctaaggaa ggtttttttt

1921 cttccctcta gtgggcgggg cagaggagtt agccaagatg tgactttgaa accctcagcg

1981 tctcagtgcc cttttgttct aaacaaagaa ttttgtaatt ggttctacca aagaaggata

2041 taatgaagtc actatgggaa aagatgggga ggagagttgt aggattctac attaattctc

2101 ttgtgccctt agcccactac ttcagaattt cctgaagaaa gcaagcctga attggttttt

2161 taaattgctt taaaaatttt ttttaactgg gttaatgctt gctgaattgg aagtgaatgt

2221 ccattccttt gcctcttttg cagatataca cttcagataa ctacaccgag gaaatgggct

2281 caggggacta tgactccatg aaggaaccct gtttccgtga agaaaatgct aatttcaata

2341 aaatcttcct gcccaccatc tactccatca tcttcttaac tggcattgtg ggcaatggat

2401 tggtcatcct ggtcatgggt taccagaaga aactgagaag catgacggac aagtacaggc

2461 tgcacctgtc agtggccgac ctcctctttg tcatcacgct tcccttctgg gcagttgatg

2521 ccgtggcaaa ctggtacttt gggaacttcc tatgcaaggc agtccatgtc atctacacag

2581 tcaacctcta cagcagtgtc ctcatcctgg ccttcatcag tctggaccgc tacctggcca

2641 tcgtccacgc caccaacagt cagaggccaa ggaagctgtt ggctgaaaag gtggtctatg

2701 ttggcgtctg gatccctgcc ctcctgctga ctattcccga cttcatcttt gccaacgtca

2761 gtgaggcaga tgacagatat atctgtgacc gcttctaccc caatgacttg tgggtggttg

2821 tgttccagtt tcagcacatc atggttggcc ttatcctgcc tggtattgtc atcctgtcct

2881 gctattgcat tatcatctcc aagctgtcac actccaaggg ccaccagaag cgcaaggccc

2941 tcaagaccac agtcatcctc atcctggctt tcttcgcctg ttggctgcct tactacattg

3001 ggatcagcat cgactccttc atcctcctgg aaatcatcaa gcaagggtgt gagtttgaga

3061 acactgtgca caagtggatt tccatcaccg aggccctagc tttcttccac tgttgtctga

3121 accccatcct ctatgctttc cttggagcca aatttaaaac ctctgcccag cacgcactca

3181 cctctgtgag cagagggtcc agcctcaaga tcctctccaa aggaaagcga ggtggacatt

3241 catctgtttc cactgagtct gagtcttcaa gttttcactc cagctaacac agatgtaaaa

3301 gacttttttt tatacgataa ataacttttt tttaagttac acatttttca gatataaaag

3361 actgaccaat attgtacagt ttttattgct tgttggattt ttgtcttgtg tttctttagt

3421 ttttgtgaag tttaattgac ttatttatat aaattttttt tgtttcatat tgatgtgtgt

3481 ctaggcagga cctgtggcca agttcttagt tgctgtatgt ctcgtggtag gactgtagaa

3541 aagggaactg aacattccag agcgtgtagt gaatcacgta aagctagaaa tgatccccag

3601 ctgtttatgc atagataatc tctccattcc cgtggaacgt ttttcctgtt cttaagacgt

3661 gattttgctg tagaagatgg cacttataac caaagcccaa agtggtatag aaatgctggt

3721 ttttcagttt tcaggagtgg gttgatttca gcacctacag tgtacagtct tgtattaagt

3781 tgttaataaa agtacatgtt aaactta

As used herein, "overexpression of CAMKK 1" means an amount of CAMKK 1 expression product (mRNA or protein) in the cell, organ or tissue of the patient that is greater than what is normally present in the organ or tissue of the patient.

As used herein, "overexpression of SDF-1" means an amount of SDF-1 expression product (mRNA or protein) in the cell, organ or tissue of the patient that is greater than what is normally present in the organ or tissue of the patient.

The term "exosome" is well-known in the art and refers to extracellular vesicles released from cells upon fusion of an intermediate endocytic compartment, the multivesicular body with the plasma membrane. See for example Edgar JR (2016) BMC Biol. 13; 14:46.

The term "exogenous" in reference to a nucleic acid or protein refers to a polynucleotide or polypeptide sequence that has been artificially introduced into a cell, cell-derived vesicles, exosomes, microvesicle, or combination thereof. There may be an endogenous nucleic acid or protein having the same or substantially similar sequence as that of the polynucleotide or polypeptide encoding the exogenous nucleic acid or protein in the cell-derived vesicles. For example, a mesenchymal stem cell can be genetically modified to overexpress a CAMKK1-encoding polynucleotide. It is contemplated that a purified population of cell-derived vesicles isolated from the culture media collected from MSCs genetically modified to overexpress CAMKK1 would contain higher levels of CAMKK1 as compared to cell-derived vesicles isolated from MSCs that have not been modified to overexpress a CAMKKl-encoding polynucleotide.

A "composition" is also intended to encompass a combination of active agent and another carrier, e.g., compound or composition, inert (for example, a detectable agent or label) or active, such as an adjuvant, diluent, binder, stabilizer, buffers, salts, lipophilic solvents, preservative, adjuvant or the like. Carriers also include biocompatible scaffolds, pharmaceutical excipients and additives proteins, peptides, amino acids, lipids, and carbohydrates (e.g., sugars, including monosaccharides, di-, tri-, tetra-, and oligosaccharides; derivatized sugars such as alditols, aldonic acids, esterified sugars and the like; and polysaccharides or sugar polymers), which can be present singly or in combination, comprising alone or in combination 1-99.99% by weight or volume. Exemplary protein excipients include serum albumin such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. Carbohydrate excipients are also intended within the scope of this disclosure, examples of which include but are not limited to monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol.

"Substantially homogeneous" describes a population of cells in which more than about 50%, or alternatively more than about 60 %, or alternatively more than 70 %, or alternatively more than 75 %, or alternatively more than 80%, or alternatively more than 85 %, or alternatively more than 90%, or alternatively, more than 95 %, of the cells are of the same or similar phenotype. Phenotype can be determined by a pre-selected cell surface marker or other marker.

A "biocompatible matrix" refers to a scaffold or matrix for tissue-engineering purposes with the ability to perform as a substrate that will support the appropriate cellular activity to generate the desired tissue, including the facilitation of molecular and mechanical signaling systems, without eliciting any undesirable effect in those cells or inducing any undesirable local or systemic responses in the eventual host. In other embodiments, a biocompatible scaffold is a precursor to an implantable device which has the ability to perform its intended function, with the desired degree of incorporation in the host, without eliciting an undesirable local or systemic effects in the host. Biocompatible scaffolds are described in U.S. Patent No. 6,993,406 and 6,103,255.

As used herein and known to the skilled artisan, a "marker" is a receptor or protein expressed by the cell or internal to the cell which can be used as an identifying and/or distinguishing factor. If the marker is noted as ("+"), the marker is positively expressed. If the marker is noted as ("-"), the marker is absent or not expressed. Variable expression of markers is also used, such as "high" and "low" and relative terms.

A "precursor" or "progenitor cell" intends to mean cells that have a capacity to differentiate into a specific type of cell. A progenitor cell may be a stem cell. A progenitor cell may also be more specific than a stem cell. A progenitor cell may be unipotent or multipotent. Compared to adult stem cells, a progenitor cell may be in a later stage of cell differentiation. An example of progenitor cell includes, without limitation, a progenitor nerve cell.

A population of cells intends a collection of more than one cell that is identical (clonal) or non-identical in phenotype and/or genotype.

The term effective amount refers to a concentration or amount of a reagent or composition, such as a composition as described herein, cell population or other agent, that is effective for producing an intended result, including cell growth and/or differentiation in vitro or in vivo, or for the treatment of a neurodegenerative condition as described herein. It will be appreciated that the number of cells to be administered will vary depending on the specifics of the disorder to be treated, including but not limited to size or total volume/surface area to be treated, as well as proximity of the site of administration to the location of the region to be treated, among other factors familiar to the medicinal biologist.

The terms effective period (or time) and effective conditions refer to a period of time or other controllable conditions (e.g., temperature, humidity for in vitro methods), necessary or preferred for an agent or composition to achieve its intended result, e.g., the differentiation of cells to a pre-determined cell type.

The term patient or subject refers to animals, including mammals, such as bovines, canines, felines, ovines, equines, preferably humans, who are treated with the pharmaceutical compositions or in accordance with the methods described herein.

The term pharmaceutically acceptable carrier (or medium), which may be used interchangeably with the term biologically compatible carrier or medium, refers to reagents, cells, compounds, materials, compositions, and/or dosage forms that are not only compatible with the cells and other agents to be administered therapeutically, but also are, within the scope of sound medical judgment, suitable for use in contact with the tissues of human beings and animals without excessive toxicity, irritation, allergic response, or other complication commensurate with a reasonable benefit/risk ratio. Pharmaceutically acceptable carriers suitable for use in the present disclosure include liquids, semi-solid (e.g., gels) and solid materials (e.g., cell scaffolds and matrices, tubes sheets and other such materials as known in the art and described in greater detail herein). These semi-solid and solid materials may be designed to resist degradation within the body (non-biodegradable) or they may be designed to degrade within the body (biodegradable, bioerodable). A biodegradable material may further be bioresorbable or bioabsorbable, i.e., it may be dissolved and absorbed into bodily fluids (water-soluble implants are one example), or degraded and ultimately eliminated from the body, either by conversion into other materials or breakdown and elimination through natural pathways.

A "control" is an alternative subject or sample used in an experiment for comparison purpose. A control can be "positive" or "negative". For example, where the purpose of the experiment is to determine a correlation of an altered expression level of a gene with a particular phenotype, it is generally preferable to use a positive control (a sample from a subject, carrying such alteration and exhibiting the desired phenotype), and a negative control (a subject or a sample from a subject lacking the altered expression or phenotype). Additionally, when the purpose of the experiment is to determine if an agent effects the differentiation of a stem cell, it is preferable to use a positive control (a sample with an aspect that is known to affect differentiation) and a negative control (an agent known to not have an affect or a sample with no agent added).

The terms autologous transfer, autologous transplantation, autograft and the like refer to treatments wherein the cell donor is also the recipient of the cell replacement therapy. The terms allogeneic transfer, allogeneic transplantation, allograft and the like refer to treatments wherein the cell donor is of the same species as the recipient of the cell replacement therapy, but is not the same individual. A cell transfer in which the donor's cells and have been histocompatibly matched with a recipient is sometimes referred to as a syngeneic transfer. The terms xenogeneic transfer, xenogeneic transplantation, xenograft and the like refer to treatments wherein the cell donor is of a different species than the recipient of the cell replacement therapy.

"Host cell" refers not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

"Treating" or "treatment" of a disease includes: (1) preventing the disease, i.e., causing the clinical symptoms of the disease not to develop in a patient that may be predisposed to the disease but does not yet experience or display symptoms of the disease; (2) inhibiting the disease, i.e., arresting or reducing the development of the disease or its clinical symptoms; or (3) relieving the disease, i.e., causing regression of the disease or its clinical symptoms. In one aspect, the treatment excludes prophylaxis.

To treat cardiomyopathy and related disorders intends the alleviation of symptoms and related clinical endpoints of these diseases or disorders, that include for example, a myocardial disorder in which heart muscle is structurally and functionally abnormal. The vast majority of cardiomyopathy is extrinsic (external causes) and of these, the ischemic form is the most common. The terms include ischemic cardiomyopathy which can refer to remodeled and hypertrophied myocardium as a result of inadequate oxygen delivery to the myocardium, with coronary artery disease being the most common cause.

The term "cardiomyopathy" is also sometimes used to specifically describe non-ischemic forms of cardiomyopathy, often with intrinsic/idiopathic causes. Non-ischemic cardiomyopathy is associated with abnormalities of the genes encoding sarcomeric proteins, the commonest types of which are hypertrophic cardiomyopathy and dilated cardiomyopathy, and combinations of myocardial hypertrophy and/or fibrosis occur in these diseases. Other types of non-ischemic cardiomyopathy include restrictive cardiomyopathy, arrhythmogenic right ventricular cardiomyopathy, endomyocardial fibrosis, and unclassified cardiomyopathy which includes left ventricular non-compaction (LVNC) and Takotsubo cardiomyopathy. Additional examples include arrhythmogenic right ventricular dysplasia (arrhythmogenic right ventricular cardiomyopathy); atrial stand still (atrial cardiomyopathy with heart block); cirrhotic cardiomyopathy; congenital cataract - hypertrophic cardiomyopathy, mitochondrial myopathy; dilated cardiomyopathy; dilated cardiomyopathy- hypergonadotropic hypogonadism; dilated cardiomyopathy with ataxia; early-onset myopathy with fatal cardiomyopathy; encephalopathy - hypertrophic cardiomyopathy - renal tubular disease; familial dilated cardiomyopathy; familial dilated cardiomyopathy with conduction defect due to LMNA mutation; familial hypertrophic cardiomyopathy; familial hypertrophic cardiomyopathy (familial hypertrophic obstructive cardiomyopathy); familial isolated arrhythmogenic right ventricular dysplasia (familial isolated arrhythmogenic right ventricular cardiomyopathy); familial isolated arrhythmogenic right ventricular dysplasia (familial isolated arrhythmogenic ventricular cardiomyopathy); familial isolated arrhythmogenic ventricular dysplasia. biventricular form (familial isolated arrhythmogenic ventricular cardiomyopathy, biventricular form); familial isolated arrhythmogenic ventricular dysplasia, left dominant form (familial isolated arrhythmogenic ventricular cardiomyopathy, left dominant form); familial isolated arrhythmogenic ventricular dysplasia, right dominant form (familial isolated arrhythmogenic ventricular cardiomyopathy, classic form); familial isolated arrhythmogenic ventricular dysplasia, right dominant form (familial isolated arrhythmogenic ventricular cardiomyopathy, right dominant form); familial isolated dilated cardiomyopathy; familial isolated dilated cardiomyopathy (familial or idiopathic dilated cardiomyopathy); familial isolated hypertrophic cardiomyopathy; familial isolated hypertrophic cardiomyopathy (familial isolated hypertrophic obstructive cardiomyopathy); Familial isolated hypertrophic cardiomyopathy (familial or idiopathic hypertrophic obstructive cardiomyopathy); familial isolated hypertrophic cardiomyopathy (Hypertrophic obstructive cardiomyopathy); familial isolated hypertrophic cardiomyopathy (Primitive hypertrophic obstructive cardiomyopathy); familial isolated restrictive cardiomyopathy; familial isolated restrictive cardiomyopathy (Familial or idiopathic restrictive cardiomyopathy); familial restrictive cardiomyopathy; familial restrictive cardiomyopathy types 1, 2 or 3; Heart-hand syndrome; hypertrophic cardiomyopathy due to intensive athletic training; Leigh syndrome with cardiomyopathy; Lipoatrophy with diabetes, leukomelanodermic papules, liver steatosis, and hypertrophic cardiomyopathy; Lysosomal disease with hypertrophic cardiomyopathy; Lysosomal disease with restrictive cardiomyopathy; Maternally-inherited cardiomyopathy and hearing loss; and Maternally-inherited cardiomyopathy and hearing loss. Additional conditions are known in the art and described, for example, in US Serial No. 14/893,920.

Non-limiting examples of associated disorders include for example, secondary cardiomyopathies that may overlap with the forms of cardiomyopathy described above and include metabolic storage disease causes (e.g. amyloidosis, haemochromatosis), inflammatory causes (e.g. Chagas disease), endocrine causes (e.g. diabetic cardiomyopathy, hyperthyroidism, acromegaly), toxicity causes (e.g. anthracycline chemotherapy, alcohol), neuromuscular (e.g. muscular dystrophy) and nutritional diseases (obesity related). These can be either ischemic or non-ischemic, inherited or acquired (secondary) in various combinations.

"To promote tissue healing" intends to heal or reduce the effect of any tissue injury, such as by mechanical (e.g., a puncture wound), chemical or electrical stress. The tissue can be internal or external (the external skin of a patient or subject).

The term "suffering" as it related to the term "treatment" refers to a patient or individual who has been diagnosed with or is predisposed to infection or a disease incident to infection. A patient may also be referred to being "at risk of suffering" from a disease because of active or latent infection. This patient has not yet developed characteristic disease pathology.

The term administration shall include without limitation, administration by oral, parenteral (e.g., intramuscular, intraperitoneal, intravenous, ICV, intracisternal injection or infusion, subcutaneous injection, or implant), by inhalation spray nasal, vaginal, rectal, sublingual, urethral (e.g., urethral suppository) or topical routes of administration (e.g., gel, ointment, cream, aerosol, etc.) and can be formulated, alone or together, in suitable dosage unit formulations containing conventional non-toxic pharmaceutically acceptable carriers, adjuvants, excipients, and vehicles appropriate for each route of administration. The disclosure is not limited by the route of administration, the formulation or dosing schedule.

### Modes for Carrying Out the Disclosure

### Recombinant Stem Cells

Disclosed herein are recombinant stem cells that overexpresses one or both of:
a. a CAMKK1 polypeptide or an equivalent thereof; or
b. an SDF-1 polypeptide or an equivalent thereof, in the presence of CXCR4 polypeptide expression.
In one aspect, the overexpression is by expression of a CAMKK1 polypeptide that comprises, or alternatively consists essentially of, or yet further consists of SEQ ID NO: 1 or 2, or by expression of an SDF-1 polypeptide that comprises, or alternatively consists essentially of, or yet further consists of SEQ ID NO: 3 or 4, and the CXCR4 polypeptide comprises, or alternatively consists essentially of, or yet further consists of SEQ ID NO: 5, or an equivalent of each thereof. Some stem cells such as mesenchymal stem cells or CD34+ stem cells express CXCR4, therefore, in another aspect, the stem cell of this disclosure is mesenchymal stem cell, a CD34+ cell or CD34+ stem cell, or a progenitor of each thereof.

In another aspect, the stem cell is selected from the group of an embryonic stem cell, an iPSC, or a somatic stem cell. In a further aspect, the stem cell comprises, or alternatively consists essentially of, or yet further consists of a polynucleotide that encodes CAMKK1 polypeptide or its equivalent in an amount effective to induce exosome production, secretion and/or function. The stem cell of this disclosure can comprises, or alternatively consists essentially of, or yet further consists of one or more polynucleotides that encodes CXCR4 and SDF-1, in an amount effective to induces exosome production, secretion and/or function. The polypeptides may be expressed by one or polynucleotides encoding the CAMKK1, SDF-1 and/or CXCR4 polypeptides, or the equivalent of each thereof, that is/are optionally comprised within a vector. The stem cell can be a mammalian stem cell such as a human stem cell. The vector can be a viral vector or a plasmid. Non-limiting examples of plasmid vectors include pHIV, pLVX, pLKO.1, pLemiR, pLV, pUC, pGEM, pSP73, pSP72, pSP64, pBR322 and pCCL plasmids.

This disclosure provides an isolated stem cell or a population of stem cells, comprising, or alternatively consisting essentially of, or yet further consisting of, isolated, exogenously added polynucleotides, viral particles, vectors that encode a CAMKK1 polypeptide or protein and/or an SDF-1 polypeptide or protein, or an equivalent of each thereof. The polynucleotides are introduced into the cells to overexpress the CAMKK1 and/or SDF-1 protein or polypeptide or their equivalents. The polynucleotides can encode a mammalian polypeptide or protein, e.g., a murine, a canine, a feline, an equine, or a human for example, and the species of the polypeptide can be the same or different than the species of the cell. In one aspect, the polypeptide and the cell are from the same species, e.g., human.

The stem cell can be embryonic, an induced pluripotent stem cell (iPSC) or a somatic (adult) and can be of any particular type, including for example, adipose-derived, hematopoietic, mesenchymal, or neuronal. The cells can be from a cultured cell line available from a vendor or primary cells isolated from a subject. In one aspect, the stem cell is a mesenchymal stem cell, a human stem cell and the polynucleotide(s) encode human polypeptides and proteins.

In one aspect, the stem cell populations are substantially homogeneous or clonal. They can be autologous or allogeneic to the subject being treated when used in therapy.

In another aspect, the stem cell is of any type or any species, e.g., a mammalian stem cell such as a human stem cell.

A non-limiting example of the CAMKK1 polypeptide or protein comprises, or alternatively consists essentially of, yet further consists of the sequence of SEQ ID NOS: 1 or 2, or their equivalent. Non-limiting examples of equivalents are provided herein. A non-limiting example of the polynucleotide encoding the SDF-1 polypeptide or protein comprises, or alternatively consists essentially of, yet further consists of the sequence of 3, or its equivalent or a polynucleotide encoding the polypeptide of SEQ ID NO: 4 or its equivalent.

These polynucleotides in the stem cells can further comprise, or alternatively consist essentially of, or yet further consist of, a promoter and/or an enhancer sequences operatively linked to the polynucleotides. These polynucleotides can be further contained within a vector, e.g., a prokaryotic or a eukaryotic vector, such as a plasmid or a viral vector. Non-limiting examples of plasmid vectors include pHIV, pLVX, pLKO.1, pLemiR, pLV, pUC, pGEM, pSP73, pSP72, pSP64, pBR322 and pCCL plasmids.

The promoter can be a constitutive or an inducible promoter that regulates expression of the polynucleotide(s). The polynucleotide(s) can further comprise a detectable label and/or a suicide gene and a promoter that regulates expression of the suicide gene. In a related embodiment, the suicide gene is the thymidine kinase gene. In a further embodiment, the polynucleotide(s) encoding the SDF-1 and/or CAMKK1 proteins or polypeptides and the suicide gene are regulated by one promoter. In a further aspect, the promoter that regulates expression of the suicide gene is a constitutive promoter.

Overexpression of CAMKK1 in the stem cell can be achieved by administering a polynucleotide encoding wild type CAMKK1 protein to the cell, e.g., wherein the wild type CAMKK 1 protein is set forth in SEQ ID NO:1. In other aspects, the increase in the level of CAMKK 1 in the cell can be achieved by administering a polynucleotide encoding a constitutively active CAMKK 1. The constitutively active CAMKK 1 can comprise a CAMKK 1-413 truncation, e.g., as set forth in SEQ ID NO:2. Alternatively, equivalents thereof can be provided. Non-limiting examples of equivalents include the constitutively active CAMKK1 can comprise a T108A mutant, a S459A mutant, or a T 108A/S459A mutant CAMKK 1. The increase in the level of CAMKK 1 in the cell can be achieved by administering a polynucleotide encoding a T108A mutant CAMKK 1 to the cell. The increase in the level of CAMKK 1 in the cell also can be achieved by administering a polynucleotide encoding a S459A mutant CAMKK 1 to the cell. The increase in the level of CAMKK 1 in the cell can be achieved by administering a to the cell polynucleotide encoding a T108A/S459A mutant CAMKK 1 to the cell. This mutant is one example of an equivalent to wild type CAMKK1.

Overexpression of SDF-1 in the stem cell can be achieved by administering a polynucleotide encoding wild type SDF-1 protein to the cell, e.g., wherein the wild type SDF-1 protein is set forth in SEQ ID NO: 4 or its equivalent. In other aspects, the increase in the level of SDF-1 in the cell can be achieved by administering a polynucleotide encoding a constitutively active SDF-1 polypeptide or protein such as for example the polynucleotide of SEQ ID NO: 3 or its equivalent.

In a related embodiment, the biological equivalent of the SDF-1 and/or CAMKK1 polynucleotides comprises a nucleic acid that hybridizes under conditions of high stringency to the complement of SEQ ID NO: 3 or the polynucleotides encoding SEQ ID NOS: 1 or 2. In another embodiment, the biological equivalent thereof comprises a nucleic acid having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to the complements or the coding strands of the polynucleotides.

The isolated cells described herein can be any of a cell of a species of the group of: murine, rats, rabbit, simians, bovines, ovine, porcine, canines, feline, farm animals, sport animals, pets, equine, and primate, and in particular a human cell. In one embodiment, the cell is a stem cell. In a related embodiment, the isolated cell is a mesenchymal stem cell. Also provided is a population of expanded stem cells having this phenotype, and the cell can be substantially homogeneous for that phenotype. In one aspect, the cells are at least 70 %, or alternatively at least 75%, or alternatively at least 80 % or alternatively at least 85 %, or alternatively at least 90 %, or alternatively at least 95 %, or alternatively at least 97 % homogenous for that phenotype.

In certain embodiments, the isolated cell as described herein comprises a certain level of the SDF-1 and/or CAMKK1 protein or polypeptides. The level can be achieved by selecting an appropriate promoter and/or enhancer that produces the desirable level of protein or by using an inducible system that regulates the amount of protein produced. These promoters and inducible systems have previously been described. In one embodiment, the isolated cell comprises, or alternatively consists essentially of, or yet further, consists of at least about 5 x10⁻⁶ ng of polypeptide or protein. In further embodiments, the isolated cell comprises, or alternatively consists essentially of, or yet further, consists of at least about 1 x10⁻⁷ ng, about 3 x10⁻⁷ ng, about 5 x10⁻⁷ ng, about 7 x10⁻⁷ ng, about 9 x10⁻⁷ ng, about 1 x10⁻⁶ ng, about 2 x10⁻⁶ ng, about 3 x10⁻⁶ ng, about 4 x10⁻⁶ ng, about 6 x10⁻⁶ ng, about 7 x10⁻⁶ ng, about 8 x10⁻⁶ ng, about 9 x10⁻⁶ ng, about 10 x10⁻⁶ ng, about 12 x10⁻⁶ ng, about 14 x10⁻⁶ ng, about 16 x10⁻⁶ ng, about 18 x10⁻⁶ ng, about 20 x10⁻⁶ ng, about 25 x10⁻⁶ ng, about 30 x10⁻⁶ ng, about 35 x10⁻⁶ ng, about 40 x10⁻⁶ ng, about 45 x10⁻⁶ ng, about 50 x10⁻⁶ ng, about 55 x10⁻⁶ ng, about 60 x10⁻⁶ ng, about 65 x10⁻⁶ ng, about 70 x10⁻⁶ ng, about 75 x10⁻⁶ ng, about 80 x10⁻⁶ ng, about 85 x10⁻⁶ ng, about 90 x10⁻⁶ ng, about 95 x10⁻⁶ ng, about 10 x10⁻⁵ ng, about 20 x10⁻⁵ ng, about 30 x10⁻⁵ ng, about 40 x10⁻⁵ ng, about 50 x10⁻⁵ ng, about 60 x10⁻⁵ ng, about 70 x10⁻⁵ ng, about 80 x10⁻⁵ ng, or about 90 x10⁻⁵ ng of the polypeptide or protein.

Vectors are convenient to introduce the polynucleotides into the stem cells. In one aspect, the term "vector" intends a recombinant vector that retains the ability to infect and transduce non-dividing and/or slowly-dividing cells and integrate into the target cell's genome. In several aspects, the vector is derived from or based on a wild-type virus or plasmid. In further aspects, the vector is derived from or based on a wild-type adenovirus, adeno-associated virus (AAV), or lentivirus. Examples of such, include without limitation, human immunodeficiency virus (HIV), equine infectious anemia virus (EIAV), simian immunodeficiency virus (SIV) and feline immunodeficiency virus (FIV). Alternatively, it is contemplated that other retrovirus can be used as a basis for a vector backbone such murine leukemia virus (MLV). It will be evident that a viral vector according to the disclosure need not be confined to the components of a particular virus. The viral vector may comprise components derived from two or more different viruses, and may also comprise synthetic components. Vector components can be manipulated to obtain desired characteristics; such as target cell specificity.

The recombinant vectors of this disclosure can be derived from primates and non-primates. Examples of primate lentiviruses include the human immunodeficiency virus (HIV), the causative agent of human acquired immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anemia virus (EIAV) and the more recently described feline immunodeficiency virus (FIV) and bovine immunodeficiency virus (BIV). Prior art recombinant lentiviral vectors are known in the art, e.g., see US Patent Nos. 6,924,123; 7,056,699; 7,07,993; 7,419,829 and 7,442,551, incorporated herein by reference.

U.S. Patent No. 6,924,123 discloses that certain retroviral sequence facilitate integration into the target cell genome. This patent teaches that each retroviral genome comprises genes called gag, pol and env which code for virion proteins and enzymes. These genes are flanked at both ends by regions called long terminal repeats (LTRs). The LTRs are responsible for proviral integration, and transcription. They also serve as enhancer-promoter sequences. In other words, the LTRs can control the expression of the viral genes. Encapsidation of the retroviral RNAs occurs by virtue of a psi sequence located at the 5' end of the viral genome. The LTRs themselves are identical sequences that can be divided into three elements, which are called U3, R and U5. U3 is derived from the sequence unique to the 3' end of the RNA. R is derived from a sequence repeated at both ends of the RNA, and U5 is derived from the sequence unique to the 5' end of the RNA. The sizes of the three elements can vary considerably among different retroviruses. For the viral genome and the site of poly (A) addition (termination) is at the boundary between R and U5 in the right hand side LTR. U3 contains most of the transcriptional control elements of the provirus, which include the promoter and multiple enhancer sequences responsive to cellular and in some cases, viral transcriptional activator proteins.

With regard to the structural genes gag, pol and env themselves, gag encodes the internal structural protein of the virus. Gag protein is proteolytically processed into the mature proteins MA (matrix), CA (capsid) and NC (nucleocapsid). The pol gene encodes the reverse transcriptase (RT), which contains DNA polymerase, associated RNase H and integrase (IN), which mediate replication of the genome.

For the production of viral vector particles, the vector RNA genome is expressed from a DNA construct encoding it, in a host cell. The components of the particles not encoded by the vector genome are provided in trans by additional nucleic acid sequences (the "packaging system", which usually includes either or both of the gag/pol and env genes) expressed in the host cell. The set of sequences required for the production of the viral vector particles may be introduced into the host cell by transient transfection, or they may be integrated into the host cell genome, or they may be provided in a mixture of ways. The techniques involved are known to those skilled in the art.

Viral vectors for use in this disclosure include, but are not limited to Invitrogen's pLenti series versions 4, 6, and 6.2 "ViraPower" system. Manufactured by Lentigen Corp.; pHIV-7-GFP, lab generated and used by the City of Hope Research Institute; "Lenti-X" lentiviral vector, pLVX, manufactured by Clontech; pLKO.1-puro, manufactured by Sigma-Aldrich; pLemiR, manufactured by Open Biosystems; and pLV, lab generated and used by Charité Medical School, Institute of Virology (CBF), Berlin, Germany.

Non-viral vectors may include a plasmid that comprises a heterologous polynucleotide capable of being delivered to a target cell, either in vitro, in vivo or ex-vivo. Non-limiting examples of plasmid vectors include pHIV, pLVX, pLKO.1, pLemiR, pLV, pUC, pGEM, pSP73, pSP72, pSP64, pBR322 and pCCL plasmids. The heterologous polynucleotide can comprise a sequence of interest and can be operably linked to one or more regulatory elements and may control the transcription of the nucleic acid sequence of interest. As used herein, a vector need not be capable of replication in the ultimate target cell or subject.

In one embodiment, the additional regulatory elements are promoters, enhancer and/or promoter/enhancer combinations. The promoter that regulates expression of the nucleic acid encoding the protein or polypeptide can be a constitutive promoter. In one aspect, the promoter that regulates the expression of the suicide gene is a constitutive promoter. Non-limiting examples of constitutive promoters include SFFV, CMV, PKG, MDNU3, SV40, Efla, UBC, and CAGG. In one aspect, the enhancer is a Woodchuck post-regulatory element ("WPRE") (see, e.g., Zufferey, R. et al. (1999) J. Virol. 73(4):2886-2992). The enhancer element can be downstream of the promoter and gene just before the 3' LTR. However, the enhancer can be in any location and is not limited to the 3'LTR.

Promoters useful in this disclosure can be constitutive or inducible. Some examples of promoters include T3 promoter, T7 promoter, SP6 promoter, SV40 early promoter, mouse mammary tumor virus LTR promoter, adenovirus major late promoter, herpes simplex virus promoter, and the CMV promoter. In one embodiment, the promoter that regulates expression of the tetracycline activator protein is a constitutive promoter. In other embodiments, the promoter is an inducible promoter, a tissue specific promoter, or a promoter that regulates expression temporally. In one embodiment, the promoter is a phosphoglycerate kinase promoter (PGK).

In one embodiment, the vector further comprises, or alternatively consists essentially of, or yet further consists of a suicide gene and a promoter that regulates expression of the suicide gene. A suicide gene is one that allows for the negative selection of the cells. In the methods described herein, a suicide gene is used as a safety system, allowing the cells expressing the gene to be killed by introduction of a selective agent. This is desirable in case the recombinant gene causes a mutation leading to uncontrolled cell growth. A number of suicide gene systems have been identified, including the herpes simplex virus thymidine kinase (tk or TK) gene, the cytosine deaminase gene, the varicella-zoster virus thymidine kinase gene, the nitroreductase gene, the Escherichia coli gpt gene, and the E. coli Deo gene (also see, for example, Yazawa K, Fisher W E, Brunicardi F C: Current progress in suicide gene therapy for cancer. World J. Surg. 2002 July; 26(7):783-9). In one embodiment, the suicide gene is the thymidine kinase (TK) gene. In one aspect, the TK gene is a wild-type TK gene. In other aspect, the TK gene is a mutated form of the gene, e.g., sr23tk. Cells expressing the TK protein can be killed using ganciclovir. In another embodiment, the nucleic acid encoding the tetracycline activator protein and the suicide gene are regulated by one promoter.

In one aspect, the promoter that regulates expression of the suicide gene is a constitutive promoter. Nucleic acids encoding different proteins may be regulated by the same promoter, produce one mRNA, and yet still result in the production of two different proteins. This can be accomplished in a variety of mechanisms. For example, a protein cleavage site can be encoded in nucleic acids between the nucleic acids encoding for the proteins. In this instance, one mRNA is produced that encodes for both proteins and the protein cleavage site. Following mRNA transcription, the mRNA is translated into a chimeric polypeptide with both proteins linked by a protease cleavage site. The protein may then be cleaved by a protease that recognizes the cleavage site. The protease may be one that is endogenously expressed or exogenously expressed from a nucleic acid transferred into the cell by gene transfer methods. Accordingly, in one embodiment, the vector further comprises, or alternatively consists essentially of, or yet further consists of a protease cleavage site between the suicide gene and the nucleic acid encoding the tetracycline activator protein. In one embodiment, the protease cleavage site is the 2A protease cleavage site. An IRES or Internal Ribosome Entry Site may also be used to produce two proteins from the same promoter. In this instance, nucleic acids encoding an IRES are cloned between the nucleic acids encoding each of the proteins. One mRNA is transcribed that encodes for both proteins, but the IRES site allows for separate translation of both proteins. Thus, two different proteins are produced by one mRNA.

In a further aspect, the vector further comprises a marker or detectable label such as a gene encoding an enhanced green fluorescent protein (EGFP), red fluorescence protein (RFP), green fluorescent protein (GFP) and yellow fluorescent protein (YFP) or the like. These are commercially available and described in the technical art.

Genes may be delivered to the cell by a variety of mechanisms commonly known to those of skill in the art. Viral constructs can be delivered through the production of a virus in a suitable host cell. Virus is then harvested from the host cell and contacted with the target cell. Viral and non-viral vectors capable of expressing genes of interest can be delivered to a targeted cell via DNA/liposome complexes, micelles and targeted viral protein-DNA complexes. Liposomes that also comprise a targeting antibody or fragment thereof can be used in the methods of this disclosure. In addition to the delivery of polynucleotides to a cell or cell population, direct introduction of the proteins described herein to the cell or cell population can be done by the non-limiting technique of protein transfection, alternatively culturing conditions that can enhance the expression and/or promote the activity of the proteins of this disclosure are other non-limiting techniques.

Other methods of delivering vectors encoding genes of the current disclosure include but are not limited to, calcium phosphate transfection, DEAE-dextran transfection, electroporation, microinjection, protoplast fusion, or liposome-mediated transfection.

### Cell-derived Vesicles

Cell-derived vesicles, also referred to as extracellular vesicles, are membrane surrounded structures that are released by cells in vitro and in vivo. Extracellular vesicles can contain proteins, lipids, and nucleic acids and can mediate intercellular communication between different cells, including different cell types, in the body. Two types of extracellular vesicles are exosomes and microvesicles. Exosomes are small lipid-bound, cellularly secreted vesicles that mediate intercellular communication via cell-to-cell transport of proteins and RNA (El Andaloussi, S. et al. (2013) Nature Reviews: Drug Discovery 12(5):347-357). Exosomes range in size from approximately 30 nm to about 200 nm. Exosomes are released from a cell by fusion of multivesicular endosomes (MVE) with the plasma membrane. Microvesicles, on the other hand, are released from a cell upon direct budding from the plasma membrane (PM). Microvesicles are typically larger than exosomes and range from approximately 100 nm to 1 µm.

Cell-derived vesicles (e.g., exosomes and/or microvesicles) can be isolated from eukaryotic cells. Non-limiting examples of cells that cell-derived vesicles can be isolated from include stem cells. Non-limiting examples of such stem cells include adult stem cells, embryonic stem cells, embryonic-like stem cells, neural stem cells, or induced pluripotent stem cells. In some embodiments, the stem cell is an adult stem cell that is optionally a mesenchymal stem cell or CD34+ cell or CD34+ stem cell

The stem cells of the present disclosure are modified, for example, by genetic modification. In some embodiments, the cells are modified to express at least one exogenous nucleic acid and/or at least one exogenous protein. The modification may be a transient modification. In other embodiments, the modification may be a stable modification. For example, the stem cell may be modified by transducing an effective amount of a vector comprising, or alternatively consisting essentially of, or yet further consisting of the polynucleotide encoding the CAMKK1 protein or polypeptide, SDF-1 protein or polypeptide and/or the CXCR4 protein or polypeptide or its equivalent. Non-limiting examples of vectors include plasmid and viral vectors. It is contemplated that by modifying the cells prior to collection of the cell-derived vesicles released by the modified cells, one will collect exosomes containing different amounts and types of proteins, lipids, and nucleic acids as compared to unmodified cells. Any method for cellular modification known to one of skill in the art can be used to modify the cells.

The cells of the present disclosure can be cultured in any culture media known to those of skill in the art. For example, the cell culture media can comprise between 5% - 40% fetal bovine serum (FBS), preferably approximately 20% FBS; between 0.5% - 5% L-glutamine, preferably approximately 1% L-glutamine; and between 0.5% - 1% penicillin and streptomycin (Penn-strep), preferably approximately 1% penn-strep, in a basal media. In some embodiments, at least a portion of the FBS is substituted with a serum replacement, for example, a platelet lysate (e.g., human platelet lysate (hPL)). In some embodiments, the amount of serum replacement (e.g., hPL) in the culture media is between 1% - 20%. In some embodiments, the cells are cultured in the absence of FBS. In other embodiments, the cells are cultured in the presence of high levels of serum, for example, 30% serum, 40% serum, 50% serum, or 60% serum.

The cells of the present disclosure can be cultured under any conditions known to those in the field. Also provided herein are methods to induce exosome secretion and/or function in a stem cell, the method comprising, or alternatively consisting essentially of, or yet further consisting of, upregulating the expression of CAMKK1 polynucleotide or protein or an equivalent of each thereof in the stem cell. In one aspect, the overexpression of CAMKK1 is in the presence of Akt expression or optionally overexpression. Yet further provided is a method to induce exosome secretion and function in a stem cell, comprising, or alternatively consisting essentially of, or yet further consisting of, upregulating the expression of an SDF-1 polypeptide or protein or an equivalent of each thereof in the stem cell, and wherein the overexpression is in the presence of CXCR4 expression or optionally overexpression. In one aspect, the overexpression of SDF-1 is in the presence of Akt expression or optionally overexpression. Also provided is a method to induce exosome secretion and function in a stem cell, comprising, or alternatively consisting essentially of, or yet further consisting of, upregulating the expression of an SDF-1 polypeptide or protein or an equivalent of each thereof in the stem cell, and wherein the overexpression is in the presence of Akt expression or optionally overexpression. In one aspect, the overexpression of SDF-1 is in the presence of CXCR4 expression or optionally overexpression. The stem cell can be embryonic, an induced pluripotent stem cell (iPSC) or a somatic (adult) stem cell and can be of any particular type, including for example, adipose, mesenchymal, or neuronal for example. In another aspect, the stem cell is a mesenchymal stem cell, a CD34+ cell or CD34+ stem cell. In another aspect, the stem cell is of any type or any species, e.g., a mammalian stem cell such as a human stem cell.

### Isolation of Extracellular Vesicles

The purified populations of cell-derived vesicles (e.g., exosomes and/or microvesicles) of the present disclosure can be isolated using any method known by those in the art and as described herein Non-limiting examples include differential centrifugation by ultracentrifugation (Théry et al. (2006) Curr. Protoc. Cell Biol. 30:3.22.1-3.22.29; Witmer et al. (2013) J. Extracellular v.2), sucrose gradient purification (Escola et al. (1998) J. Biol. Chem. 273:20121-20127), and combination filtration/concentration (Lamparski et al. (2002) J. Immunol. Methods 270:211-226).

### Formulations and Compositions

The present disclosure provides purified populations of cell-derived vesicles (e.g., exosomes and/or microvesicles). In some embodiments, the population of cell-derived vesicles is substantially homogeneous. In other embodiments, the population of cell-derived vesicles is heterogeneous.

In some embodiments, the substantially homogeneous population is a purified population where at least 90% of the cell-derived vesicles have a diameter of less than 100 nm as determined by a NanoSight LM10HS (available from Malvern Instruments Ltd, Amesbury, MA, USA).

In some embodiments, the concentration of cell-derived vesicles in the population comprises between about 0.5 micrograms and 100 micrograms of exosome and/or microvesicle protein collected per approximately 10⁶ cells as determined by DC assay (Biorad, Hercules, CA, USA). In other embodiments, the concentration of cell-derived vesicles in the population comprises between about 40 micrograms and 100 micrograms of exosome and/or microvesicle protein collected per approximately 10⁶ cells. In yet other embodiments, the concentration of cell-derived vesicles in the population comprises less than about 30 micrograms of cell-derived vesicles protein collected per approximately 10⁶ cells. In yet other embodiments, the concentration of cell-derived vesicles in the population is less than about 20 micrograms per 10⁶ cells.

The purified populations of cell-derived vesicles can be purified on the basis of average size of the cell-derived vesicles in the composition. Without being bound by theory, it is contemplated that the different sized cell-derived vesicles may contain different types and/or amounts of nucleic acids, protein, lipids, and other components. As such, it is contemplated that compositions comprising cell-derived vesicles of an average size may have a different therapeutic efficacy as compared to a composition comprising cell-derived vesicles of a different average size. In some embodiments, the average diameter of the cell-derived vesicles in the population is between about 0.1 nm and about 1000 nm. In other embodiments, the average diameter of the cell-derived vesicles in the population is between about 2 nm and about 200 nm. In other embodiments, the average diameter of the cell-derived vesicles in the population is less than 100 nm. In yet other embodiments, the average diameter of the cell-derived vesicles in the population is less than 50 nm. In still other embodiments, the average diameter of the cell-derived vesicles in the population is less than about 40 nm.

The compositions disclosed herein may further comprise a carrier, for example, a pharmaceutically acceptable carrier. In some embodiments, more than one pharmaceutically acceptable carrier can be used. Any pharmaceutically acceptable carrier known to those of skill in the art can be used.

In some embodiments, the pharmaceutically acceptable carrier is a preservative, for example, a polymeric preservative.

In some embodiments, the pharmaceutically acceptable carrier is selected from the group consisting of a polyethylene glycol (PEG)( e.g., PEG 150 Distearate), honey, a large molecular weight protein (e.g., bovine serum albumin or soy protein), polyvinyl alcohol, glyceryl monostearate, hyaluronic acid, glycerin, preferably vegetable-derived, proteins, preferably hydrolyzed proteins, (e.g., soy protein and silk protein), vasoline, citrosept, parabens, xanthan gum, i-carregaan, phytagel, Carbopol^{®} polymers, and polyvinyl pyrrolidone. In some embodiments, the stabilizer is selected from the group consisting of dextran 40, methylcellulose, gelatin, sodium sulfite, and meta sodium sulfate. In other embodiments, the stabilizer is selected from the group consisting of the DES mixture as described in the United States Patent No. 9,696,247, proteins or high molecular weight molecules, such as human serum albumin, or molecules such as dextran or poloxamer.

In one aspect, the biocompatible gelation agent is an agent that is liquid prior to application to a subject (e.g., at room temperature or colder) and becomes a gel after application to the subject (e.g., at body temperature). In one embodiment, the biocompatible gelation agent is a hydrogel.

In another aspect, disclosed herein is a composition comprising exosomes and/or microvesicles and a poloxamer wherein the composition is in a sol (liquid) phase at about 0 °C to about 20 °C and transitions a gel (solid) phase at or near the body temperature or higher, such as about 25 °C to about 40 °C, or about 30 °C to about 37 °C.

In some aspects, the pharmaceutically acceptable carrier is a pharmaceutically acceptable aqueous carrier such as water or an aqueous carrier. Examples of pharmaceutically acceptable aqueous carrier include sterile water, saline, phosphate buffered saline, aqueous hyaluronic acid, Ringer's solution, dextrose solution, Hank's solution, and other aqueous physiologically balanced salt solutions. In some embodiments, the pharmaceutically acceptable aqueous carrier is Normosol^{™}-R.

Non-aqueous pharmaceutically acceptable carriers include, fixed oils, vegetable oils such as olive oil and sesame oil, triglycerides, propylene glycol, polyethylene glycol, and injectable organic esters such as ethyl oleate can also be used.

Pharmaceutically acceptable carrier can also contain minor amounts of additives, such as substances that enhance isotonicity, chemical stability, or cellular stability. Examples of buffers include phosphate buffer, bicarbonate buffer and Tris buffer, while examples of preservatives include thimerosol, cresols, formalin and benzyl alcohol. In certain aspects, the pH can be modified depending upon the mode of administration. In some aspect, the composition has a pH in the physiological pH range, such as pH 7 to 9.

In one aspect, depending on the type of a pharmaceutically acceptable carrier used, the compositions described herein can comprise about 0.1-100%, 0.1-50%, or 0.1-30%, such as 0.1 %, 0.25 %, 0.5 %, 0.75%, 1 %, 2 %, 5 %, 7 %, 10 %, 15 %, 20 %, 25 %, 30 %, 40 %, 45 % , 50 %, 55 %, 60 %, 65 %, 70 %, 75 %, 80 %, 85 %, 90 % or 95 % of the pharmaceutically acceptable carrier used in the total weight of the composition, or any range between two of the numbers (end point inclusive).

In some embodiments, any one of the above listed pharmaceutically acceptable carriers is expressly excluded.

In some embodiments, the cell-derived vesicles described herein are frozen (e.g., snap-frozen) or freeze-dried (e.g., lyophilized) to promote stability, preserve activity and increase shelf-life. One skilled in the art would understand how to reconstitute the lyophilized product before use.

In some embodiments, the populations of cell-derived vesicles described herein are used immediately after isolation. In other embodiments, the populations of cell-derived vesicles are cryopreserved (e.g. frozen), for example, using any cryopreservation techniques well-known to those skilled in the art. In some embodiments, all or substantially of the cells and/or cellular debris are removed from the culture medium prior to cryopreservation. In some embodiments, all or substantially of the cells and/or cellular debris are removed from the culture medium after cryopreservation.

### Therapeutic Methods

Provided herein are methods to promote tissue healing, to treat tissue injury or to treat a condition related to tissue injury, comprising, or alternatively consisting essentially of, or yet further consisting of administering to a subject in need thereof an effective amount of one or both of:
a. a CAMKK1 polypeptide or an equivalent thereof; or
b. an SDF-1 polypeptide or an equivalent thereof, in the presence of CXCR4 polypeptide expression,
wherein the effective amount is an amount that induces exosome production, secretion and/or function in the subject.

In one aspect, the CAMKK 1 polypeptide can comprise, or alternatively consist essentially of, or yet further consist of SEQ ID NO: 1 or 2, or an equivalent of each thereof. In another aspect, the SDF-1 polypeptide may comprise, or alternatively consist essentially of, or yet further consist of SEQ ID NO: 3 or 4, or an equivalent of each thereof. In a further aspect, the CXRC4 polypeptide can comprise, or alternatively consist essentially of, or yet further consist of SEQ ID NO 5, or an equivalent thereof.

For the methods described above, the one or more polypeptides are administered by administration of a polynucleotide encoding the polypeptide or its equivalent. In one aspect, the one or more polypeptides are administered in a vector comprising, or alternatively consisting essentially of, or yet further consisting of the polynucleotide or the equivalent thereof. The vector can be a viral vector or a plasmid vector. The polynucleotide or its equivalent or vectors can be administered by administration of a stem cell comprising, or alternatively consisting essentially of, or yet further consisting of the polynucleotide or its equivalent. The cells can be autologous or allogeneic to the subject being treated. In one aspect, the stem cell is a mesenchymal stem cell or its progenitor. For the SDF-1 polypeptide or an equivalent thereof the administration is local to the tissue that is injured. For the CAMKK1 polypeptide or an equivalent thereof the administration is local or distal to the tissue that is injured. In another aspect, the tissue injury or to treat a condition related to tissue injury is a muscle tissue or a cardiac tissue injury or a condition related to a muscle tissue or a cardiac tissue injury. In a further aspect, the related condition comprises an ischemic condition or cardiomyopathy. The methods can be combined with other methods for such treatment as known in the art.

The methods described above may further comprise, or alternatively consist essentially of, or yet further consist of determining the level of circulating exosomes in a sample isolated from the patient prior to administration. In another aspect, the methods may further comprise, or alternatively consist essentially of, or yet further consist of determining the level of circulating exosomes in a sample isolated from the patient after administration. The effective amount can be determined from the level of circulating exosomes in the sample, either from on-site testing or historical clinical information

It is known in the art that know that ischemic conditions and inflammatory conditions can occur in a number of different organs in tissues. For example, ischemic conditions can occur in the heart, liver, kidney, brain, spine, lungs, small intestine, large intestine, and arteries. Likewise, inflammatory conditions can occur in the heart, liver, kidney, brain, spine, lungs, intestines, arteries, joints, cartilage, and skin. Accordingly, disclosed are methods of treating an ischemic or inflammatory condition in an organ or tissue of a patient, wherein said organ or tissue is heart, liver, kidney, brain, spine, lungs, small intestine, large intestine, arteries, joints, cartilage, skin, or any combination thereof. In some embodiments, the methods can be used to treat an ischemic or inflammatory condition in the heart, such as cardiomyopathy. In some embodiments, the methods can be used to treat an ischemic or inflammatory condition in the myocardium. In some embodiments, the methods can be used to treat an ischemic or inflammatory condition in the liver. In some embodiments, the methods can be used to treat an ischemic or inflammatory condition in the kidney. In some embodiments, the methods can be used to treat an ischemic or inflammatory condition in the brain. In some embodiments, the methods can be used to treat an ischemic or inflammatory condition in the spine. In some embodiments, the methods can be used to treat an ischemic or inflammatory condition in the lungs. In some embodiments, the methods can be used to treat an ischemic or inflammatory condition in the small intestine. In some embodiments, the methods can be used to treat an ischemic or inflammatory condition in the large intestine. In some embodiments, the methods can be used to treat an ischemic or inflammatory condition in the arteries. In some embodiments, the methods can be used to treat an ischemic or inflammatory condition in the joints. In some embodiments, the methods can be used to treat an ischemic or inflammatory condition in the cartilage. In some embodiments, the methods can be used to treat an ischemic or inflammatory condition in the skin. In some embodiments, the methods can be used to treat an ischemic or inflammatory condition in any of the above organs or tissues. One of skill in the art can determine when the method has achieved some clinical benefit from reduction or clinical symptoms or clinical markers associated with inflammation, tissue injury, ischemic condition and/or cardiomyopathy.

Thus, this disclosure provides a method to treat one or more of: an ischemic condition, cardiomyopathy and related disorders, or promote tissue healing, comprising, or alternatively consisting essentially of administering to a subject in need thereof an effective amount one or more of: the stem cell as described herein, the isolated culture media as described herein, the population of cells as described herein, or the compositions as described herein, wherein the cells and products prepared from the cells overexpress CAMKK1 polypeptide or protein or an equivalent thereof. The cells can be autologous or allogeneic to the subject being treated. Further provided herein are methods to treat one or more of: an ischemic condition, cardiomyopathy and related disorders or to promote tissue healing, comprising, or alternatively consisting essentially of administering to a subject in need thereof an effective amount of one or more of: the exosome population of this disclosure or the composition described herein. Administration can be local or systemic. In one aspect, the administration is distal to the site of action, such as when the site of action is the heart. In another aspect, the tissue injury or to treat a condition related to tissue injury is a muscle tissue or a cardiac tissue injury or a condition related to a muscle tissue or a cardiac tissue injury. The methods can be combined with other methods for such treatment as known in the art.

Also provided herein is a method to treat one or more of an ischemic condition or cardiomyopathy and related disorders, comprising administering to a subject in need thereof an effective amount one or more of: the stem cell as described herein, the isolated culture media as described herein, the population of cells as described herein, or the compositions as described herein, wherein the cells and products prepared from the cells overexpress SDF-1 polypeptide or protein or an equivalent thereof. Administration can be local or systemic.

The cells or conditioned media can be administered systemically, directly into the ischemic or inflamed tissue or about the periphery of the ischemic or inflamed tissue. Suitable techniques for systemic administration include enteral administration or parenteral administration (injection, infusion, or implantation). The protein, vector, cells, or conditioned media can be administered, for example, orally, epidurally, intracerebrally, intracerebroventricularly, intraarterially, intraarticularly, intracardially, intramuscularly, intralesionally, intraperitoneally, intrathecally, intravenously, subcutaneously, or any combination thereof.

The ischemic or inflammatory condition can be acute myocardial infarction, cardiomyopathy, heart failure, chronic heart failure, peripheral artery disease, stroke, liver disease, ischemic kidney disease, multiple sclerosis, traumatic brain injury, spinal cord injury, graft versus host disease (GVHD), diabetes, chronic obstructive pulmonary disease (COPD), rheumatoid arthritis, an injury from a solid organ transplant, an orthopedic injury, a cartilage disorder, a wound, or any combination thereof.

The disclosed methods can further comprise administering one or more additional regenerative therapies. Suitable additional regenerative therapies include, but are not limited to, mesenchymal stem cells derived from bone marrow, adipose tissue, placental tissue, umbilical cord, Wharton's Jelly, menstrual blood, stem cells, M2 macrophages, monocytes, or any combination thereof. The stem cells can be neural progenitor cells, endothelial progenitor cells, organ specific endogenous stem cells, or any combination thereof. In some aspects, the methods can further comprise administering neural progenitor cells. In some aspects, the methods can further comprise administering endothelial progenitor cells. In some aspects, the methods can further comprise administering endogenous stem cells, such as cardiac ckit+ cells. In some aspects, the methods can further comprise administering any combination of the above stem cells. The therapeutic methods may further comprise, or alternatively consist essentially of, or yet further consist of determining the level of circulating exosomes in a sample isolated from the patient prior to administration. In another aspect, the methods may further comprise, or alternatively consist essentially of, or yet further consist of determining the level of circulating exosomes in a sample isolated from the patient after administration. The effective amount can be determined from the level of circulating exosomes in the sample.

The administered stem cell can be embryonic, an induced pluripotent stem cell (iPSC) or a somatic (adult) and can be of any particular type, including for example, adipose, mesenchymal, or neuronal for example. In one aspect, the stem cell is a mesenchymal stem cell, a CD34+ cell or a CD34+ stem cell. In another aspect, the stem cell is of any type or any species, e.g., a mammalian stem cell such as a human stem cell Differentiated cells produced from the stem cells are further provided herein. The cell can be allogeneic or autologous to the subject being treated, e.g., a mammal such as a canine, a feline, an equine or a human. As is apparent to the skilled artisan, in most aspects, the cell, polynucleotide and treated subject are of the same species, e.g., a human cell transduced with a human gene to treat a human patient.

As known to the skilled artisan, the dosage, cell, and dosing schedule are determined by the clinical practitioner and will vary with the condition being treated, the age and gender of the subject and the species of the subject. One of skill in the art can determine when the method has been successful by noting certain clinical or sub-clinical endpoints. One of skill in the art can determine when the method has achieved some clinical benefit from reduction or clinical symptoms or clinical markers associated with inflammation, tissue injury, ischemic condition and/or cardiomyopathy. The methods can be combined with other suitable therapies as determined by the treating clinician.

The following examples are provided to illustrate and not limit this disclosure.

### Experimental 1

As shown in FIG. 1, the goal of this experiment was to determine if CAMKK1 had an effect in chronic heart failure, and how this compared to SDF-1. Based on Applicant's understanding of the mechanism of CAMKK1, the working hypothesis was that CAMKK1 would not have any effect on cardiac function, SDF-1 would, as Applicant previously had determined that the combination of SDF-1 and CAMKK1 (not shown) would be better than SDF-1 alone. The rats used in the experiment were 9 month post-anterior wall myocardial infarction; therefore, they had well developed ischemic cardiomyopathies. They were dosed with either SDF-1 plasmid or CAMKK1 plasmid by opening the chest of the rat and directly injecting the plasmid into the peri-infarct area. Echocardiography was performed at baseline (9 months after heart attack) and then as a function of time after dosing. FIG. 1 shows that SDF-1 and CAMKK1 individually had significant and equivalent effects resulting in increased ejection fraction compared to placebo. The combination of SDF-1 and CAMKK1 did not result in any greater increase in cardiac function than either of the molecules alone.

### Experiment No. 2

Prior to the conducting the work described in Experiment No. 1. Applicant had assumed that SDF-1 worked through its chemokine effect and resulted in improvement in cardiac function through the homing of tissue specific and bone marrow derived stem cells. CAMKK1 does not induce stem cell homing. That CAMKK1 and SDF-1 had equivalent effects on cardiac function, Applicant sought to define a common pathway through which SDF-1 and CAMKK1 might work. Exosomes were isolated from the conditioned media of control mesenchymal stem cells (MSC), and MSC over-expressing either SDF-1 or CAMKK1. There were 2 million MSC for each treatment. The isolated exosomes were then injected into the heart of a rat immediately after inducing an anterior wall myocardial infarction by tying off the left anterior descending coronary artery. As shown in FIG. 2, a significant effect of SDF-1 and CAMKK1 is the enhancement of exosome release and function into the conditioned media.

### Experiment No. 3

Applicant then sought to define the mechanism of the enhancement of MSC effects on CAMKK1 on cardiac function. The results (see FIG. 3) show that the enhancement of MSC effects of CAMKK1 on cardiac function requires the phosphorylation of Akt. In these studies, like above, the conditioned media of 2 million MSC were collected and injected into the heart of animals immediately after inducing an anterior wall myocardial infarction. In two treatments MSC were treated with one of two Akt inhibitors and the presence of either Akt inhibitor decreased the benefits of the CAMKK1 over-expressing cells to that of control MSC. Applicant also determined that SDF-1 requires Akt phosphorylation as well (data not shown). It is critical to note at this point that CAMKK1 is a kinase and directly phosphorylates Akt; whereas, SDF-1 phosphorylates Akt through binding of the CXCR4 receptor. Therefore, the effects of SDF-1 require an autocrine or paracrine loop to induce Akt phosphorylation, while the CAMKK1 effect is direct.

### Experiment No. 4

Experiment No. 4 evaluated the effects of direct and indirect Akt phosphorylation. Rats underwent ligation of the left anterior descending coronary artery and immediately after the chest was closed, CAMKK1 plasmid or SDF-1 plasmid or saline, was injected into the thigh of the animal. One week later, the cardiac function of the animals injected with CAMKK1 was significantly increased compared to saline, but the heart function in SDF-1 treated animals was not.

### Experiment No. 5

### Animals

Eight-week-old male Lewis rats were maintained under standard conditions in the animal facility at Northeast Ohio Medical University. All animal procedures were approved by the Institutional Animal Care and Use Committee.

### MSC Preparation

MSC were isolated from rat bone marrow of adult Lewis rats. The cells were isolated by flushing the femurs with 0.6 ml Dulbecco's modified Eagle's medium (DMEM) (Gibco, Invitrogen, http://www.invitrogen.com, Carlsbad, CA, fishersci.com). Clumps of bone marrow were gently minced with an 18-gauge needle and then centrifuged for 5 minutes at 260 g and washed with two changes of phosphate-buffered saline (Invitrogen). The washed cells were then resuspended and plated in DMEM-Low Glucose (Gibco, Invitrogen) with 10% fetal bovine serum (FBS) (Gibco, Invitrogen). The cells were incubated at 37°C, and nonadherent cells were removed by replacing the medium after 3 days. When cultures became 70% confluent, adherent cells were detached following incubation with 0.05% trypsin and 2 mM EDTA (Invitrogen) for 5 minutes. MSC cultures were then depleted of CD45+ and CD34+ cells simultaneously by negative selection using 1 µl each of primary phycoerythrin-conjugated mouse anti-rat CD45 (BD Biosciences, http://www.bdbiosciences.com, San Diego, CA) and CD34 antibodies (Santa Cruz Biotechnology, https://www.scbt.com, Santa Cruz, CA) per 10 × 6 cells using the EasySep PE selection kit (Stem Cell Technologies, Vancouver, Canada, https://www.stemcell.com/). Finally, Icam-positive cells were selected by cell sorting.

### MSC and MSC-conditioned Medium

In preparation for transplantation, MSC transfection was performed by Neon Transfection system electroporation. Applicants transfected 2 × 10⁶ cells with cDNA:CAMKK1 or siRNA:CAMKK1 or cDNA:SDF-1, CDNA AKT or siRNA:AKT, siRNA:CAMK4 with transfection efficiency of 55%-80%. Conditioned medium was generated as follows: After eleven passages, 90% confluency was noted in both the control and experimental groups. Conditioned medium was generated by feeding cells transfected with different treatments with serum-free DMEM and incubated for 48 hours. Control medium was generated by using only cells without treatment. The medium was collected and the cells were counted for normalization purposes: For each animal, Applicants used medium generated by 2 × 10⁶ cells. After removing the cellular debris, supernatants were transferred to 12-ml tubes.

### Total Exosome Isolation (from condition media MSC)

### ExoQuick^{™} precipitation

ExoQuick^{™} precipitation was carried out according to manufacturer's instructions (System Biosciences). Briefly, 500 µL of clarified MSC-CM was diluted to 5 mL in PBS and mixed with 1 mL of ExoQuick-TC^{™} solution by inverting the tube several times. The sample was incubated overnight at 4°C then centrifuged twice at 1,500 g for 30 and 5 minutes, respectively, in order to remove the supernatant. The supernatant was discarded, and the pellet was resuspended in 200 µL of PBS. The isolated exosomes were injected in five different sites at the infarct border zone immediately after the left anterior descending (LAD) ligation

### Quantification and size determination of the exosomes by Nanoparticle Tracking Analysis (NTA).

Nanoparticle tracking analysis (NTA) is a powerful characterization technique that combines the properties of both laser light scattering microscopy and Brownian motion in order to obtain size distributions of particles in liquid suspension. NanoSight instruments (Malvern, UK), which currently are the most widely used instruments for NTA in the exosomes field, are equipped with one or more lasers and an optical microscope connected to a digital camera. According to the manufacturer, NanoSight enables characterization of particles from 10-2000 nm in solution. Particles are visualized by the light they scatter upon laser illumination, and their Brownian motion is monitored. The NTA software enables sizing of single particles by tracking their mean squared displacement and thereby calculating their theoretical hydrodynamic diameter using the Stokes Einstein equation. On the basis of knowing the analyzed sample volume, NTA also allows for an estimation of particle concentration. Analysis of size distribution of homogeneous particle preparations of either same or mixed sizes by NanoSight has previously been shown to be accurate

### LAD Ligation to Induce MI

Myocardial infarction was induced in rats using a surgical microscope (LeicaMicrosystems M500) as previously described. Briefly, animals were anaesthetized with intraperitoneal ketamine and xylazine, intubated and ventilated with room air at 80 breaths/minutes using a pressure-cycled rodent ventilator (RSP1002; Kent Scientific Corporation). Sternotomy was performed and the proximal LAD was ligated with 7-0 prolene. Observed blanching and dysfunction of the anterior wall verified LAD ligation. After LAD ligation, the animals were evaluated with echocardiography and euthanized at different time points for organ harvest (heart) and staining.

### Administration of exosomes after AMI

The rats were injected with saline (n=6), Exo-CAMKK1 (n=7), Exo-SDF-1 (n=7) Exo- AKT (n=6) siRNA-CAMKK1 and AKT, CAMK4 (n=7) immediately after AMI. In all groups, the injection volume was 600 µL exosomes solution injected in five different sites at the infarct border zone immediately after the left anterior descending (LAD) ligation. Animals from each group were distributed equally amount of exosomes. The animal mortality rate was 10% for this study.

### Echocardiographic Assessment of Heart Function

Two-dimension echocardiography was performed with a 15-MHz linear array transducer interfaced with a Sequoia C256 (Acuson) as preciously described ^{XXXX} Briefly, baseline and left ventricular (LV) dimensions were quantified pre-LAD ligation as well as 2 and 6 weeks after LAD ligation, LV dimensions were quantified by digitally recorded 2D clips and M-mode images from the mid-LV just below the papillary muscles to allow for consistent measurements from the same anatomical location in different rats. Ejection fraction (EF), fractional shortening, diastolic thicknesses of the LV posterior wall, systolic thicknesses of the LV posterior wall, diastolic LV internal dimensions, and systolic LV internal dimensions were measured. Echocardiographic measurements were performed and analyzed by investigators who were blinded to the treatment and identity of the rat.

### Statistics

Data are represented as mean ± SEM. Comparisons between two groups were made with a two-tailed Student *t* test with significance defined as *p* < .05*.* Comparisons among multiple groups were made with analysis of variation (ANOVA).

### Characterization of MSC-Derived Exosomes

To investigate the effect of CAMKK1-exosomes derived MSC, Applicants precipitate exosomes from 2 million of conditioned media of control Mesenchymal Stem Cells (MSC), and MSC over-expressing CAMKK1 as described above. The isolated exosomes were then injected into the heart of a rat immediately after ligation of the LAD. **FIG. 5A****,** the data showed that overexpressing of CAMKK1 in MSC enriches the amount of exosomes (3.76 × 10⁹ ± 2.2 particles/ml, p < 0.05) higher than control group (2.7 × 10⁹ ± 4.9 particles/ml), with respect to the size, the exosomes population was homogenous. Nanoparticle Tracking Analysis estimated the size of the Exosomes between 10nm - 1000nm. FIG. 5B, exo-CAMKK1 has a significant effect of improving the cardiac function compared to Saline-control group. The notable effect of CAMKK1 is the enhancement of exosomes release and function into the conditioned media. The direct injection of exosomes isolated from MSC that over-express CAMKK1 lead to a significantly greater increase in cardiac function 2 weeks after AMI compared to that seen with exosomes from control MSC. (FIG. 5B)

### MSC effects of CAMKK1 on cardiac function requires the phosphorylation of Akt

Applicants wanted to determine the relevant phosphorylation targets of CAMKK1 on enhanced exosome function and number. Akt, CAMK1 and CAMK4 were inhibited. The data in FIG. 6 demonstrate that inhibiting Akt phosphorylation inhibited the enhanced effects of CAMKK1 over-expression on MSC derived exosomes. The data in FIG. 7 further demonstrate that down-regulating CAMK1 also inhibits the enhanced effect of CAMKK1 on MSC derived exosomes.

### Effects of CAMKK1 and SDF-1 on cardiac function in ischemic cardiomyopathy

Applicants have previously demonstrated that CAMKK1 does not induce SDF-1 and SDF-1 does not induce CAMKK1. Given the understanding of the mechanism of action of the two different molecules, Applicants hypothesized that SDF-1 and CAMKK1 would have different effects in developed ischemic cardiomyopathy. To test this hypothesis, Applicants injected DNA plasmid encoding SDF-1, CAMKK1 or both into the peri-infarct zone of rats 9 months after AMI. As seen in FIG. 8, in response to gene injection, there was an identical improvement in cardiac function between SDF-1 and CAMKK1, with no further improvement with the combination (data not shown).

### CAMKK1 and SDF-1 mediate tissue repair through exosome release

The similarities between effects of SDF-1 and CAMKK1 in ischemic cardiomyopathy led to the investigation of whether SDF-1 also mediates its effects through increasing and enhancing exosome function. To test this hypothesis, Applicants isolated exosomes from MSC that overexpressed CAMKK1 or SDF-1. As seen in FIG. 9 improvements in cardiac function (ejection fraction) 2 weeks after AMI, are seen with exosomes isolated from MSC that overexpressed CAMKK1 or SDF-1. Exosomes from CAMKK1 or SDF-1 over-expressing MSC led to a significant improvement in EF (21.8% ± 2.5, p < .01) and SDF-1 (16.8% ± 2.7, p > .05) compared with Saline control group (1.9% ± 0.2) after 2 weeks post-AMI. Applicants repeated these experiments with the CXCR4 inhibitor AMD3100 added to the cell culture media to inhibit paracrine and autocrine SDF-1 binding to its receptor. In these studies, exosomes derived from SDF-1 over-expressing MSC in the presence of AMD-3100 did not improve cardiac function 2 weeks after AMI compared to saline control (FIG. 11 and data not shown).

### Direct Effects of CAMMK1 on Exosome Generation Allows for Efficacy from Remote Gene Expression.

The above data indicate that the effects of SDF-1 require local CXCR4 expression; whereas the kinase, CAMKK1 has no known receptor and works directly through intracellular effects on AKt and CAMK4. Given the evidence by some that the infusion of exosomes leads to improved tissue repair, Applicants hypothesized that the remote injection of CAMKK1 plasmid would lead to myocardial repair, but that SDF-1 injection into remote normal tissue would not. To test this hypothesis, Applicants induced AMI by LAD ligation and injected CAMKK1 or SDF-1 plasmid into the thigh of the animal. As seen in FIG. 10, those animals that had AMI and CAMKK1 injected into their thigh demonstrated a significant increase in cardiac function compared to saline controls. SDF-1 plasmid injection into the thigh led to no significant improvement in cardiac function compared to control.

### Experimental Summary

Exosome release by stem cells is a growing area of regenerative therapies. Increasing evidence indicates that exosome release by MSCs mediate the tissue preservation and healing effects of cell therapy. Over the past two decades, Applicants have demonstrated in pre-clinical studies that the over-expression of the chemokine stromal cell-derived factor-1 (SDF-1), and more recently kinase calcium/calmodulin-dependent protein kinase kinase-1 (CAMKK1) lead to decreased myocardial infarct size, and improved cardiac function in models of acute myocardial infarction. Clinical studies have demonstrated that direct injection of SDF-1 plasmid in the peri-infarct zone leads to improved cardiac function, but only in the most severely injured hearts. Applicants disclose herein that CAMKK1 over-expression leads to identical levels of improvement in cardiac function as seen with SDF-1 over-expression. It is further demonstrated that the effects of both CAMKK1 and SDF-1 are mediated through the enhanced release and function of exosomes. This is in direct contrast to the long-held belief that SDF-1 mediates tissue repair through stem cell homing.
It is further demonstrated herein that the effects of SDF-1 require local tissue CXCR4 expression in order to generate exosome release through an autocrine or paracrine loop, whereas, the effects of CAMKK1 are direct and require phosphorylation of AKT and CAMK4. Myocardial infarction was induced in an animal model SDF-1 or CAMKK1 plasmid was injected into the thigh of the animal. CAMKK1 over-expression in the thigh led to improved cardiac function (CAMKK1 30.3% ± 1.5, p < .01); whereas, SDF-1 overexpression had no effect and SDF-1 (15.7% ± 3.7, p > .05) compared with Saline control group (12.9% ± 1.8). These data demonstrate unique pathways for the induction of the potential regenerative effects of CAMKK1 and SDF-1 over-expression; suggesting that CAMKK1 over-expression is a more direct strategy for inducing tissue repair. These differences have important therapeutic implications and help to explain previous failed trials with SDF-1. Furthermore, to the extent that the effects of cell therapy are due to SDF-1 release, these data offer mechanistic insight into why the effects of cell therapy have been shown to correlate with the degree of tissue injury, because greater tissue injury leads to greater CXCR4 expression, leading to the greater generation of exosomes in response to stem cell release of SDF-1.

### Partial Sequence Listing

SEQ ID: NO: 1
   CAMKK1 full length protein
   NM_032294 (ncbi.nlm.nih.gov/nuccore/NM_032294; last accessed on April 13, 2018) 
MEGGPAVCCQDPRAELVERVAAIDVTHLEEADGGPEPTRNGVDP
PPRARAASVIPGSTSRLLPARPSLSARKLSLQERPAGSYLEAQAGPYATGPASHISPR
AWRRPTIESHHVAISDAEDCVQLNQYKLQSEIGKGAYGVVRLAYNESEDRHYAMKVLS
KKKLLKQYGFPRRPPPRGSQAAQGGPAKQLLPLERVYQEIAILKKLDHVNVVKLIEVL
DDPAEDNLYLVFDLLRKGPVMEVPCDKPFSEEQARLYLRDVILGLEYLHCQKIVHRDI
KPSNLLLGDDGHVKIADFGVSNQFEGNDAQLSSTAGTPAFMAPEAISDSGQSFSGKAL
DVWATGVTLYCFVYGKCPFIDDFILALHRKIKNEPVVFPEEPEISEELKDLILKMLDK
NPETRIGVPDIKLHPWVTKNGEEPLPSEEEHCSVVEVTEEEVKNSVRLIPSWTTVILV
KSMLRKRSFGNPFEPQARREERSMSAPGNLLVKEGFGEGGKSPELPGVQEDEAAS
SEQ ID NO: 2
   CAMKK1 1-413 truncated polypeptide

MEGGPAVCCQDPRAELVERVAAIDVTHLEEADGGPEPTRNGVDP
PPRARAASVIPGSTSRLLPARPSLSARKLSLQERPAGSYLEAQAGPYATGPASHISPR
AWRRPTIESHHVAISDAEDCVQLNQYKLQSEIGKGAYGVVRLAYNESEDRHYAMKVLS
KKKLLKQYGFPRRPPPRGSQAAQGGPAKQLLPLERVYQEIAILKKLDHVNVVKLIEVL
DDPAEDNLYLVFDLLRKGPVMEVPCDKPFSEEQARLYLRDVILGLEYLHCQKIVHRDI
KPSNLLLGDDGHVKIADFGVSNQFEGNDAQLSSTAGTPAFMAPEAISDSGQSFSGKAL
DVWATGVTLYCFVYGKCPFIDDFILALHRKIKNEPVVFPEEPEISEELKDLILKMLDK
NPETRIGVPDIKLHPWVTKNG

### Equivalents

It is to be understood that while the disclosure has been described in conjunction with the above embodiments, that the foregoing description and examples are intended to illustrate and not limit the scope of the disclosure. Other aspects, advantages and modifications within the scope of the disclosure will be apparent to those skilled in the art to which the disclosure pertains.

In addition, where features or aspects of the disclosure are described in terms of Markush groups, those skilled in the art will recognize that the disclosure is also thereby described in terms of any individual member or subgroup of members of the Markush group.

All publications, patent applications, patents, and other references mentioned herein are expressly incorporated by reference in their entirety, to the same extent as if each were incorporated by reference individually. In case of conflict, the present specification, including definitions, will control.
1. A method to promote tissue healing, to treat tissue injury, or to treat a condition related to tissue injury, comprising administering to a subject in need thereof an effective amount of one or both of:
   a. a CAMKK1 polypeptide or an equivalent thereof; or
   b. an SDF-1 polypeptide or an equivalent thereof, in the presence of CXCR4 polypeptide expression,
   wherein the effective amount is an amount that induces exosome production, secretion and/or function in the subject.
2. The method of clause 1, wherein the CAMKK 1 polypeptide comprises SEQ ID NO: 1 or 2, or an equivalent of each thereof.
3. The method of clause 1, wherein the SDF-1 polypeptide comprises SEQ ID NO: 3 or 4, or an equivalent of each thereof.
4. The method of clause 1 or 3, wherein the CXRC4 polypeptide comprises SEQ ID NO 5, or an equivalent thereof.
5. The method of any preceding clause, wherein the one or more polypeptides are administered by administration of a polynucleotide encoding the polypeptide or its equivalent.
6. The method of clause 5, wherein the one or more polypeptides are administered in a vector comprising the polynucleotide or the equivalent thereof.
7. The method of clause 5, wherein the vector is a viral vector or a plasmid vector.
8. The method of any one of clauses 5 to 7, wherein the polynucleotide or its equivalent or vectors are administered by administration of a stem cell comprising the polynucleotide or its equivalent.
9. The method of clause 8, wherein the stem cell is a mesenchymal stem cell or its progenitor.
10. The method of clause 1b, wherein the administration is local to the tissue that is injured.
11. The method of clause 1a, wherein the administration is local or distal to the tissue that is injured.
12. The method of any preceding clause, wherein the tissue is muscle tissue or cardiac tissue.
13. The method of any preceding clause, wherein the related condition comprises an ischemic condition or cardiomyopathy.
14. The method of any preceding clause, further comprising determining the level of circulating exosomes in a sample isolated from the patient prior to administration.
15. The method of any preceding clause, further comprising determining the level of circulating exosomes in a sample isolated from the patient after administration.
16. The method of clause 14 or 15, wherein the effective amount is determined from the level of circulating exosomes in the sample.
17. A recombinant stem cell that overexpresses one or both of:
   a. a CAMKK1 polypeptide or an equivalent thereof; or
   b. an SDF-1 polypeptide or an equivalent thereof, in the presence of CXCR4 polypeptide expression.
18. The stem cell of clause 17, wherein the overexpression is by expression of a CAMKK1 polypeptide that comprises SEQ ID NO: 1 or 2, or by expression of an SDF-1 polypeptide that comprises SEQ ID NO: 3 or 4, and the CXCR4 polypeptide comprises SEQ ID NO: 5, or an equivalent of each thereof.
19. The stem cell of clause 17 or 18, wherein the stem cell is mesenchymal stem cell, a CD34+ stem cell or a progenitor of each thereof.
20. The stem cell of clause 18, wherein the stem cell is selected from the group of an embryonic stem cell, an iPSC, or a somatic stem cell.
21. The stem cell of clause any one of clauses 17 to 20, wherein the stem cell comprises a polynucleotide that encodes CAMKK1 polypeptide or its equivalent in an amount effective to induce exosome production, secretion and/or function.
22. The stem cell of any one of clauses 17 to 21, wherein the stem cell comprises one or more polynucleotides that encodes CXCR4 and SDF-1, in an amount effective to induces exosome production, secretion and/or function.
23. The stem cell of any one of clauses 17-22, wherein the stem cell is a mammalian stem cell.
24. The stem cell of clause 23, wherein the mammalian stem cell is a human stem cell.
25. The stem cell of any one of clauses 17-24, wherein the polypeptides are expressed by one or polynucleotides encoding the CAMKK1, SDF-1 and/or CXCR4 polypeptides, or the equivalent of each thereof, that is/are optionally comprised within a vector.
26. The stem cell of any one of clauses 21, 22 or 25, wherein the vector is a viral vector or a plasmid.
27. A method to produce therapeutic exosomes comprising growing the stem cell of any one of clauses 17 to 26 under conditions that promote exosome production and isolating the exosomes produced therefrom.
28. Isolated cell culture media produced by the stem cell of any one of clauses 17 to 26.
29. A population of exosomes produced by the cells of any one of clauses 17-26.
30. A population of exosomes isolated from the cell culture media of clause 28.
31. A composition comprising a carrier and one or more of the group of: an isolated stem cell of any one of clauses 17 to 26, the isolated cell culture media of clause 26, or the population of clause 29 or 30.
32. The composition of clause 31, wherein the carrier is a pharmaceutically acceptable carrier or a biocompatible matrix.
33. The composition of clause 31 or 32, further comprising a preservative, a stabilizer, and/or a cryoprotectant.
34. A lyophilized composition comprising the population of exosomes of clause 29 or 30.
35. The composition of clause 34, further comprising a cryoprotectant.
36. A method to treat one or more of: an ischemic condition, cardiomyopathy and related disorders or to promote tissue healing, comprising administering to a subject in need thereof an effective amount of one or more of: the exosome population of clause 29 or 30; or the composition of clause 32 or 33.
37. The method of clause 36, wherein the administration is local or systemic.
38. The method of clause 36, wherein the administration is distal to the site of the tissue.
39. The method of any one of clauses 36 to 38, wherein the tissue is muscle tissue or cardiac tissue.
40. The method of any one of clauses 36 to 39, further comprising determining the level of circulating exosomes in a sample isolated from the patient prior to administration.
41. The method of any one of clauses 36 to 39, further comprising determining the level of circulating exosomes in a sample isolated from the patient after administration.
42. The method of clause 40 or 41, wherein the effective amount is determined from the level of circulating exosomes in the sample.
43. A method to induce exosome secretion and function in a cell, comprising upregulating the expression of a CAMKK1 polypeptide or protein or an equivalent of thereof in a cell.
44. The method of clause 43, wherein the cell is a stem cell.
45. The method of clause 44, wherein the stem cell is selected from the group of: an embryonic stem cell, an iPSC or a somatic stem cell.
46. The method of clause 44, wherein the stem cell is a mesenchymal stem cell or its progenitor.
47. The method of any of clauses 44 to 46, wherein the stem cell is a mammalian stem cell.
48. The method of clause 47, wherein the mammalian stem cell is a human stem cell.
49. The method of any one of clauses 44 to 48, wherein the CAMKK1 protein or polypeptide comprises the sequence of SEQ ID NO: 1 or 2, or an equivalent of each thereof, and the expression of CAMKK1 is upregulated by transducing the stem cell with an effective amount of a polynucleotide encoding the CAMKK1 protein or polypeptide or its equivalent.
50. The method of clause 49, wherein the polynucleotide further comprises a detectable label.
51. The method of clause 49 or 50, wherein the polynucleotide further comprises a promoter and/or an enhancer sequences operatively linked to the polynucleotide or its equivalent.
52. The method of clause 50 or 51, wherein the polynucleotide further comprises a vector.
53. The method of clause 52, wherein the vector is a prokaryotic or a eukaryotic vector.
54. The method of clause 53, wherein the prokaryotic vector is a plasmid.
55. The method of clause 53, wherein the eukaryotic vector is selected from the group of: an adenoviral vector, an adeno-associated viral vector or a retroviral vector.
56. The method of any one of clauses 43-55, further comprising culturing the cell for an effective amount of time for the cells to export exosomes into the culture media and then optionally separating the cells from the culture media.
57. The method of clause 56, further comprising isolating a population of exosomes from the culture media or the stem cells.
58. A method to induce exosome secretion and function in a stem cell, comprising upregulating the expression of SDF-1 protein or an equivalent of thereof in the stem cell, and wherein the expression is in the presence of CXCR4 expression.
59. The method of clause 58, wherein the stem cell is selected from the group of: an embryonic stem cell, an iPSC or a somatic stem cell.
60. The method of clause 59, wherein the stem cell is a mesenchymal stem cell stem cell or its progenitor.
61. The method of any of clauses 58-60, wherein the stem cell is a mammalian stem cell.
62. The method of clause 61, wherein the mammalian stem cell is a human stem cell.
63. The method of any one of clauses 58-62, wherein the SDF-1 protein comprises the sequence of SEQ ID NO: 4 or 5, or an equivalent of each thereof and the expression of SDF-1 is upregulated by transducing the stem cell with an effective amount of a polynucleotide encoding the SDF-1 protein or its equivalent.
64. The method of clause 62 or 63, wherein the SDF-1 protein is expressed by a polynucleotide that further comprises a promoter and/or an enhancer sequences operatively linked to the polynucleotide or its equivalent.
65. The method of clause 64, wherein the polynucleotide further comprises a vector.
66. The method of clause 65, wherein the vector is a prokaryotic or a eukaryotic vector.
67. The method of clause 66, wherein the prokaryotic vector is a plasmid.
68. The method of clause 66, wherein the eukaryotic vector is selected from the group of: an adenoviral vector, an adeno-associated viral vector or a retroviral vector.
69. The method of clause 67 or 68, further comprising culturing the cell for an effective amount of time for the cells to export exosomes into the culture media and then optionally separating the cells from the culture media.
70. The method of clause 69, further comprising isolating a population of exosomes from the culture media or the stem cells.

## Claims

1. A composition for use in a method of treating a cardiomyopathy or an ischemic condition in a subject, the composition comprising an effective amount of one or both of:
a) a vector comprising a polynucleotide encoding a CAMKK1 polypeptide; or
b) a vector comprising a polynucleotide encoding an SDF-1 polypeptide.

2. The composition for use according to claim 1, wherein the effective amount is an amount that induces exosome production, secretion and/or function in the subject.

3. The composition for use according to claim 1, wherein the CAMKK1 polypeptide is administered distal to a tissue that is injured.

4. The composition for use according to claim 1, wherein the SDF-1 polypeptide is administered local to a tissue that is injured.

5. The composition for use according to claim 4, wherein the tissue that is injured is muscle tissue or cardiac tissue.

6. The composition for use according to claim 1, wherein the composition comprises the vector comprising the polynucleotide encoding the CAMKK1 polypeptide and the vector comprising the polynucleotide encoding the SDF-1 polypeptide.

7. The composition for use according to claim 1, wherein the composition comprises the vector comprising the polynucleotide encoding the CAMKK1 polypeptide.

8. The composition for use according to claim 7, wherein the CAMKK1 polypeptide comprises SEQ ID NO: 1 or 2, or an equivalent of each thereof.

9. The composition for use according to claim 1, wherein the composition comprises the vector comprising the polynucleotide encoding the SDF-1 polypeptide.

10. The composition for use according to claim 9, wherein the SDF-1 polypeptide comprises SEQ ID NO: 4 or an equivalent thereof.

11. The composition for use according to claim 1, further comprising a vector comprising a polynucleotide encoding a C-X-C chemokine receptor type 4 (CXCR4) polypeptide.

12. The composition for use according to claim 1, wherein the vector comprising the polynucleotide encoding the SDF-1 polypeptide further comprises a polynucleotide encoding a C-X-C chemokine receptor type 4 (CXCR4) polypeptide.

13. The composition for use according to claim 1, wherein the vector is a viral vector or a plasmid.

14. The composition for use according to claim 1, wherein the composition is formulated for intravenous or intramuscular administration.
